# EUROPEAN PATENT SPECIFICATION

(11) **EP 0 749 967 B1**
(45) Date of publication and mention of the grant of the patent: **22.01.2003**
(21) Application number: 96110050.0
(22) Date of filing: 21.06.1996
(51) Int. Cl.: C07D 279/02, C07D 275/06, C07D 417/06, C07D 513/10, A61K 31/54

(54) **Substituted benzothiazine derivative**
Substituiertes Benzothiazinderivat
Dérivé de benzothiazine substitué

(30) Priority: 22.06.1995 JP 17797695
(43) Date of publication of application: 27.12.1996
(73) Proprietor: SUNTORY LIMITED, Kita-ku, Osaka-shi, Osaka 530 (JP)
(72) Inventor: Mizuno, Akira, Kyoto-shi, Kyoto 612 (JP); Shibata, Makoto, Ashikaga-shi, Tochigi 326 (JP); Iwamori, Tomoe, Ibaraki-shi, Osaka 567 (JP); Inomata, Norio, Mino-shi, Osaka 562 (JP)
(74) Representative: VOSSIUS & PARTNER

(56) References cited:
- EP-A- 0 433 149
- EP-A- 0 686 632
- WO-A-93/16073
- US-A- 3 284 450

## Description

This invention relates to novel substituted benzothiazine derivatives. More specifically, this invention is concerned with substituted benzothiazine derivatives and salts thereof, said derivatives and salts being useful for the prevention or treatment of ischemic heart diseases such as angina pectoris, arrhythmia, myocardial infarction, congestive heart failure and post-PTCA restenosis, cerebrovascular disturbances such as cerebral infarction and cerebral sequalae after subarachnoid hemorrhage, and/or peripheral circulatory disturbances such as arteriosclerosis obliterans, Raynaud disease, Buerger disease and thrombophlebitis; their preparation process; and pharmaceuticals comprising them as effective ingredients.

Serotonin is a compound contained abundantly in platelets, which are a blood component, and in a central nervous system, it acts as a neurotransmitter. In platelets, it is released upon stimulation by thromboxane A₂, ADP, collagen or the like and synergistically acts on various platelet aggregation factors or vasoconstrictors through activation of serotonin-2 receptors in the platelets and vascular smooth muscle cells, thereby inducing strong platelet aggregation and vasoconstriction [P.M. Vanhoutte, "Journal of Cardiovascular Pharmacology", Vol. 17 (Supple. 5), S6-S12 (1991)].

Serotonin is also known to potentiate proliferation of vascular smooth muscle cells [S. Araki et al., "Atherosclerosis", Vol. 83, p29-p34(1990)]. It has been considered that, particularly when endothelial cells are injured as in arteriosclerosis or myocardial infarction, the vasoconstricting action and thrombus forming action of serotonin are exasperated, thereby reducing or even stopping blood supply to myocardial, cerebral and peripheral organs [P. Golino et al., "The New England Journal of Medicine", Vol. 324, No. 10, p641-p648(1991), Y. Takiguchi et al., "Thrombosis and Haemostasis", Vol. 68(4), p460-p463(1992), A.S. Weyrich et al., "American Journal of Physiology", Vol. 263, H349-H358(1992)].

Being attracted by such actions of serotonin or serotonin-2 receptors, various attempts are now under way to use a serotonin-2 receptor antagonist as a pharmaceutical for ischemic diseases of the heart, the brain and peripheral tissues.

Ketanserin which has therapeutically been used as a hypotensive drug is known as a compound having antagonistic action against a serotonin-2 receptor. Ketanserin has strong antagonistic action against a sympathetic nerve α₁ receptor and also against histamine-1 and dopamine receptors in addition to antagonistic action against serotonin-2 receptors so that there is the potential problem of developing excessive hypotensive action, neuroleptic action or the like when used for the treatment of ischemic heart disease or peripheral circulatory disturbance. Ketanserin is therefore not preferred.

In addition, several compounds led by sarpogrelate are known to have serotonin-2 receptor antagonistic action. They, however, are accompanied with problems in the potency, the selectivity against other receptors, toxicity, side effects or the like. Thus, there remains still much room for improvements.

In view of the foregoing circumstances, it has been found that specific substituted benzothiazine derivatives have strong serotonin-2 receptor antagonistic action, is excellent in the selectivity of a serotonin-2 receptor in the antagonistic action against various receptors, particularly in the selectivity to a serotonin-2 receptor in the antagonistic action against α₁ receptor, and have low toxicity, leading to the completion of the present invention.

The present invention has been completed based on the above described findings and a first object of the present invention is to provide a benzothiazine derivative as defined in the attached claims set.

Another object of the present invention is to provide a preparation process of the substituted benzothiazine derivative (I) or its salt.

A further object of the present invention is to provide a pharmaceutical such as a therapeutic for circulatory diseases or the like, said pharmaceutical containing the substituted benzothiazine derivative (I) or respective compounds wherein G represents an ethylene group and both of X₂ and X₃ are oxygen atoms or pharmacologically-acceptable salts thereof as an effective ingredient.

The substituted benzothiazine derivatives (I) and their salts according to the present invention have strong serotonin-2 blocking action, have excellent selectivity to this action against α₁ blocking action and have high safety.

In the substituted benzothiazine derivatives (I) of the present invention, R₂ represents branched or linear C₁₋₄ alkyl groups such as methyl and ethyl, C₆₋₁₄ aryl groups such as phenyl and naphtyl and C₇₋₂₂ aralkyl groups such as benzyl and phenethyl, each of which may be substituted by one or more of halogen atoms such as fluorine, chlorine and bromine; C₁₋₄ alkyl groups such as methyl and ethyl; and/or C₁₋₄ alkoxy groups such as methoxy and ethoxy. In this case, preferred examples of group R₂X₁- include methoxy, methylthio, ethoxy and ethylthio groups.

Preferred examples of the following group: 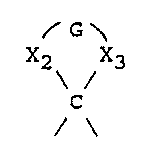 includes groups represented by the following formulas: 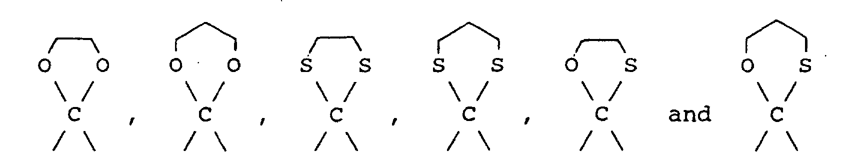 in which one or more of the hydrogen atoms may be substituted by a corresponding number of halogen atoms such as fluorine, chlorine and bromine; alkyl groups, preferably C₁₋₄ alkyl groups such as methyl and ethyl; aryl groups, preferably C₆₋₁₄ aryl groups such as phenyl and naphtyl; aralkyl groups, preferably C₇₋₂₂ aralkyl groups such as benzyl and phenethyl; and/or alkylidene groups, preferably C₁₋₄ alkylidene groups such as methylidene and ethylidene,
provided that G is not an ethylene group if both of X₂ and X₃ are oxygen atoms.

Specifically preferred examples of the group Z include the following groups: 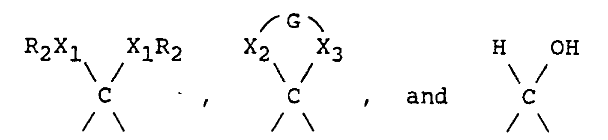 wherein G, R₁, R₂, R₄, X₁, X₂ and X₃ have the same meanings as defined above.

Q₁ represents a hydrogen atom; a hydroxyl group; halogen atoms such as fluorine, chlorine and bromine; branched or linear C₁₋₄ alkyl groups such as methyl and ethyl; branched or linear C₁₋₄ alkoxy groups such as methoxy and ethoxy . Of these Q₁, particularly preferred include a hydrogen atom, a methoxy group, a chlorine atom and the like.

Q₂ represents a hydroxyl group; halogen atoms such as fluorine, chlorine and bromine; branched or linear C₁₋₄ alkyl groups such as methyl and ethyl; branched or linear C₁₋₄ alkoxy groups such as methoxy and ethoxy. Of these Q₂, particularly preferred include a methoxy group, a chlorine atom and the like.

Further, preferred examples of substituting positions and combinations of Q₁ and Q₂ include combinations of a hydrogen atom as Q₁ and 5-hydroxy, 5-chloro, 5-bromo, 5-methyl, 5-ethyl, 5-n-propyl, 5-isopropyl, 5-n-butyl, 5-s-butyl, 5-methoxy, 5-ethoxy, 5-n-propoxy, 5-isopropoxy, 6-hydroxy, 6-fluoro, 6-chloro, 6-methyl, 6-ethyl, 6-n-propyl, 6-methoxy, 6-ethoxy, 6-n-propoxy, 7-hydroxy, 7-fluoro, 7-chloro, 7-methyl, 7-ethyl, 7-n-propyl, 7-methoxy, 7-ethoxy, 7-n-propoxy, 8-hydroxy, 8-fluoro, 8-chloro, 8-methyl, 8-ethyl, 8-n-propyl, 8-methoxy, 8-ethoxy and 8-n-propoxy as Q₂; and also combinations of 5,7-dihydroxy, 6,7-dichloro, 5,8-dimethyl, 6,8-dimethyl, 5,6-dimethoxy, 5,7-dimethoxy, 5,8-dimethoxy and 6,7-dimethoxy as Q₁ and Q₂.

Group A represents branched or linear C₂₋₁₀ alkylene groups such as ethylene, trimethylene, tetramethylene, pentamethylene and octamethylene. Among them, ethylene, trimethylene and tetramethylene groups are particularly preferred.

The group, which is represented by the following formula: 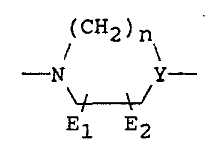 wherein E₁, E₂, Y and n have the same meanings as defined above, is a heterocyclic group led by a pyrrolidine, piperidine, piperazine or homopiperazine group.

When the heterocyclic group is a group derived from piperazine or homopiperazine, preferably a piperazine group, m stands preferably for 0.

Group D represents a phenyl group or pyridyl, pyrimidinyl, benzisothiazolyl, benzisoxazolyl and indolyl groups with one or more hydrogen atoms thereof having been optionally substituted.

Substituent groups for the aromatic hydrocarbon groups and aromatic heterocyclic groups include halogen atoms such as fluorine, chlorine and bromine; C₁₋₄ alkyl groups such as methyl and ethyl; C₁₋₄ alkoxy groups such as methoxy and ethoxy; cyano group; nitro group; carboxyl group; alkoxycarbonyl group (the number of carbons in the alcohol moiety ranges from 1 to 6); lower alkylsulfonylamino groups (the number of carbon atoms in the alkyl moiety ranges from 1 to 4); a carbamoyl group; and a hydroxyl group.

Of these illustrative groups represented by D, preferred are phenyl groups unsubstituted or substituted by one or more of halogen atoms, alkoxy groups and hydroxyl groups, benzisothiazolyl groups unsubstituted or substituted by one or more halogen atoms, benzisoxazolyl groups unsubstituted or substituted by one or more halogen atoms, and indazolyl groups unsubstituted or substituted by one or more halogen atoms. Particularly preferred are phenyl groups unsubstituted or substituted by one or more of fluorine atoms, methoxy groups and hydroxyl groups.

Many of the compounds (I) according to the present invention have isomers. It is to be noted that these isomers and mixtures thereof are all embraced by the present invention.

Various processes can be employed for the preparation of the substituted benzothiazine derivatives (I) according to the present invention. It is however preferred to prepare the benzothiazine derivatives, for example, by any one of the following processes.

### Process 1:

Among the substituted benzothiazine derivatives (I), each of compounds (Ib) in which Z is represented by one of the following formulas: 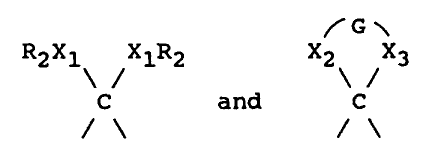 can be synthesized in accordance with any of the processes shown by the following schemes.

(a) Following the below-described reaction scheme, a compound represented by the formula (XXIII) is treated with an acid into a compound represented by the formula (XV), followed by the reaction with the a compound represented by the formula (XXVII) or the formula (XXVIII) into a compound represented by the formula (XXIV). The compound (XXIV) is reacted with a compound represented by the formula (III) so that the former compound is converted to a compound represented by the formula (XXV). The compound (XXV) is reacted further with a nitrogen-containing compound represented by formula (V) or a salt thereof, whereby a compound (Ib) is obtained.

As an alternative process for obtaining the compound (XXV) from the compound (XXIII), it is possible to adopt a process in which, after the compound (III) is reacted to the compound (XXIII), the reaction product is subjected to deacetalization, followed by the reaction with the compound (XXVII) or the compound (XXVIII). 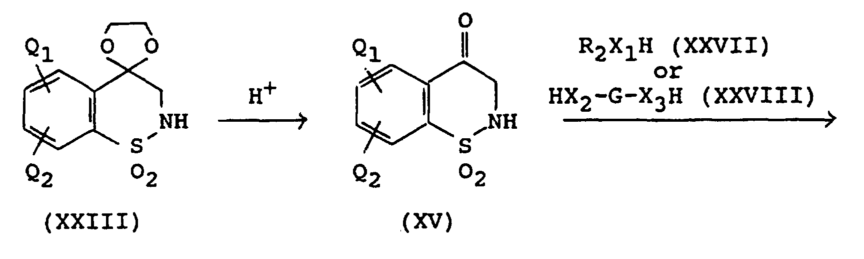 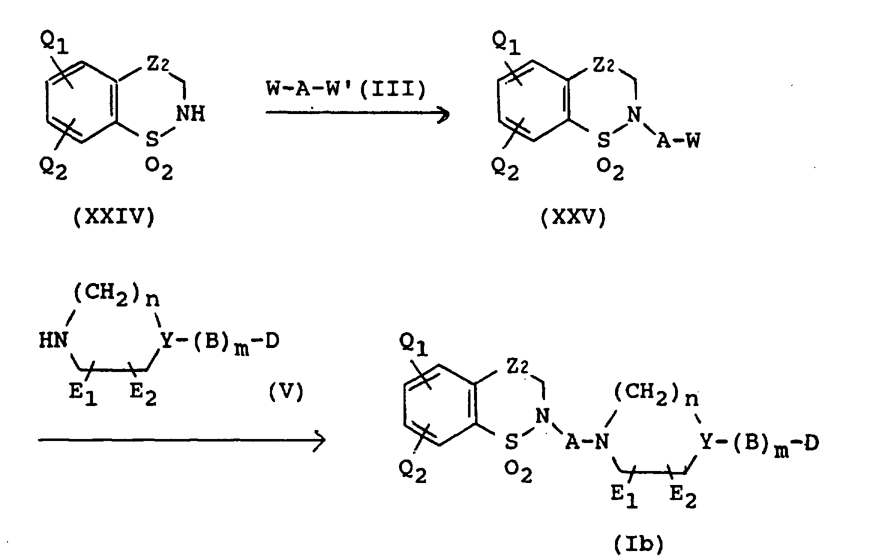 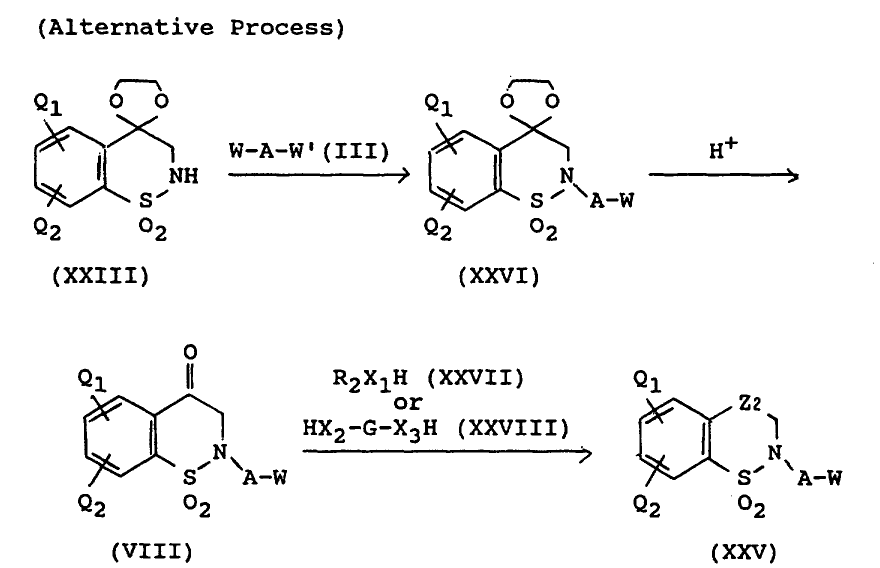 wherein A, B, D, E₁, E₂, Q₁, Q₂, Y, m and n have the same meanings as defined above; Z₂ represents one of the following groups: 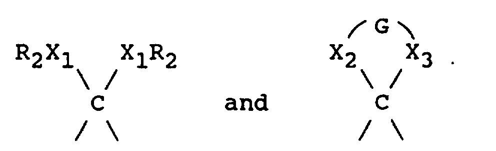 in which G, R₂, X₁, X₂ and X₃ have the same meanings as defined above; and W and W' may be the same or different and individually represents a substituent easily replaceable with an amino group.

In the above reactions, the conversion from the compound (XXIII) to the compound (XV) or that from the compound (XXVI) to the compound (VIII) in the alternative process can be effected choosing a suitable process described T.W. Green in "Protective Groups in Organic Synthesis", John Wiley & Sons, Inc. or the like. As a typical example, a process in which an acid such as hydrochloric acid or acetic acid is caused to act on the compound (XXIII) or the compound (XXVI) can be mentioned.

Further, the conversion from the compound (XV) to the compound (XXIV) or that from the compound (VIII) to the compound (XXV) in the alternative process can be effected choosing an adequate process described by T.W. Green in "Protective Groups in Organic Synthesis", John Wiley & Sons, Inc. or the like. As a typical example, a process in which ethylene glycol, ethanedithiol, ethanethiol or the like is caused to act on the compound (XV) in the presence of an acid can be mentioned.

In the above reactions, the conversion from the compound (XXIV) to the compound (XXV) or that from the compound (XXIII) to the compound (XXVI) in the alternative process can be effected by causing the compound (III) to act on the compound (XXIV) or the compound (XXIII) after treating the compound (XXIV) or the compound (XXIII) with an inorganic base or an organic base, or by causing the compound (III) to act on the compound (XXIV) or the compound (XXIII) in the presence of such a base.

Examples of group W or W' of the compound (III), which is an eliminative substituent and is easily replaceable with an amino group, include halogen atoms such as chlorine and bromine, alkylsulfonyloxy groups such as methanesulfonyloxy and arylsulfonyloxy groups such as p-toluenesulfonyloxy.

On the other hand, exemplary inorganic or organic bases include sodium hydride, potassium hydride, sodium carbonate, potassium carbonate, triethylamine and potassium t-butoxide. Further, illustrative solvents useful for the above reaction include tetrahydrofuran, dioxane, dimethylformamide, dimethylsulfoxide, acetonitrile, N-methylpyrrolidone, acetone, 2-butanone and toluene. The reaction is conducted at -78°C to reflux temperature.

To prepare the compound (Ib) by reacting the thus-obtained compound (XXV) with the nitrogen-containing compound (V), it is only necessary to react the nitrogen-containing compound (V) or an organic acid salt or inorganic acid salt thereof with the compound (XXV), optionally together with an organic base such as triethylamine, pyridine, collidine, 1,8-diazabicyclo[5.4.0]undec-7-en (DBU) or potassium t-butoxide or an inorganic base such as potassium carbonate, sodium carbonate, sodium hydrogencarbonate, sodium hydroxide or sodium hydride, optionally after adding an alkali iodide such as potassium iodide or sodium iodide, at 0°C to 150°C in the solvent exemplified above or a solvent such as methanol, ethanol, propanol or butanol.

Examples of the nitrogen-containing compound (V) include 1-phenylpiperazine, 1-(2-fluorophenyl)piperazine, 1-(3-fluorophenyl)piperazine, 1-(4-fluorophenyl)piperazine, 1-(4-hydroxyphenyl)piperazine, 1-(2-chlorophenyl)piperazine, 1-(3-chlorophenyl)piperazine, 1-(4-chlorophenyl)piperazine, 1-(2-methoxyphenyl)piperazine, 1-(3-methoxyphenyl)piperazine, 1-(4-methoxyphenyl)piperazine, 1-(4-methanesulfonamidophenyl)piperazine, 1-(4-cyanophenyl)piperazine, 1-(4-carbamoylphenyl)piperazine, 1-(4-methoxycarbonylphenyl)piperazine, 1-(2-pyridyl)piperazine, 1-(2-pyrimidinyl)piperazine, 1-benzylpiperazine, l-diphenylmethylpiperazine, 1-cinnamylpiperazine, 1-benzoylpiperazine, 1-(4-benzyloxybenzoyl)piperazine, 1-(4-hydroxybenzoyl)piperazine, 1-(2-furoyl)piperazine, 1-(1,2-benzisooxazol-3-yl)piperazine, 1-(1,2-benzisothiazol-3-yl)piperazine, 4-phenylpiperidine, 4-benzylpiperidine, α,α-bis(4-fluorophenyl)-4-piperidinemethanol, 4-(4-fluorobenzoyl)piperidine, 4-benzoylpiperidine, 4-(4-methoxybenzoyl)piperidine, 4-(4-chlorobenzoyl)piperidine, 3-(4-fluorobenzoyl)piperidine, 4-(6-fluoro-1,2-benzisoxazol-3-yl)piperidine, 4-(6-fluoro-1,2-benzisothiazol-3-yl)piperidine, 4-(6-fluoro-lH-indazol-3-yl)piperidine, 3-benzoylpyrrolidine, 3-(4-fluorobenzoyl)pyrrolidine, 4-(4-fluorophenoxy)piperidine, 4-[(4-fluorophenyl)thio]piperidine, 4-[(4-fluorophenyl)sulfinyl]piperidine, 4-[(4-fluorophenyl)sulfonyl]piperidine, 4-[bis(4-fluorophenyl)methylene]piperidine and 4-(4-fluorobenzoyl)piperidine ethylene acetal. They are all either known compounds or compounds which can be readily prepared by a known process or a process similar to the known process.

In the above reactions, the compound (XXIII) employed as the starting material can be prepared using as a raw material a saccharin derivative represented by the formula (XXIX) in accordance with the following reaction scheme: 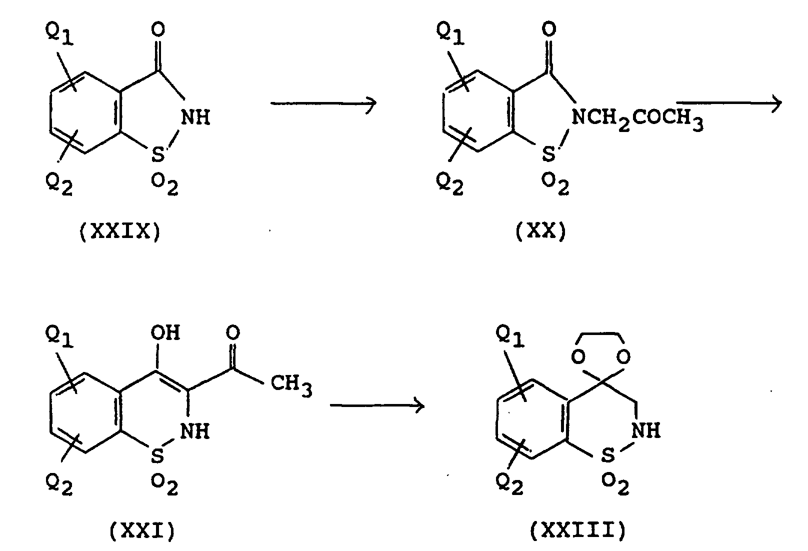 wherein Q₁ and Q₂ have the same meanings as defined above.

In the above reactions, the conversion from the compound (XXIX) to the compound (XX) can be conducted using the process proposed by E. Eckenroth in Ber., **29**, 329 (1896), and the conversion from the compound (XX) to the compound (XXI) can be conducted using the process proposed by H. Zinnes in J. Org. Chem., **30**, 2241 (1965). Further, the conversion from the compound (XXI) to the conversion (XXIII) can be performed using the process proposed by H. Zinnes in J. Org. Chem., **31**, 162 (1966).

Substituted saccharins (XXIX) usable as starting materials in the above reactions are either known compounds or compounds which can be readily prepared by a known process or a process similar to the known process. For example, preparation processes for saccharins substituted by one or more hydroxyl groups are disclosed in JP-A-56-166181, JP-A-61-215382, etc., while preparation processes for saccharins substituted by one or more halogen atoms such as fluorine, chlorine and bromine atoms were proposed, for example, by W. Davies in J. Chem. Soc., **119**(I), 876 (1921), by F. Becke et al. in Liebigs Ann. Chem., **729**, 146 (1969), by J.G. Lombardino in J. Org., Chem., **36**, 1843 (1971) and by Nitta et al. in Yakugaku Zasshi, **84**, 496 (1964) and are also disclosed in JP-A-52-71464, JP-A-56-166181, JP-A-5-194444, etc.

Further, preparation processes for saccharins substituted by one or more alkyl groups such as methyl groups were proposed by J.G. Lombardino in J. Org. Chem., **36**, 1843 (1971) and are disclosed in JP-A-52-71464, JP-A-61-215382, JP-A-5-194444, etc. and preparation processes for saccharins substituted by one or more alkoxy groups such as methoxy groups were proposed by J.G. Lombardino in J. Org. Chem., **36**, 1843 (1971) and are disclosed in JP-A-52-71464, JP-A-56-166181, JP-A-61-263961, JP-A-5-194444, etc.

Accordingly, substituted saccharins (XXIII) containing desired substituent groups as Q₁ and Q₂ can be obtained by these processes or by processes derived with reference to such processes.

(b) The target compound can be obtained by causing a nitrogen-containing compound represented by the formula (VI) or a salt thereof to act on the compound represented by the formula (XXIV) in accordance with the following reaction scheme: 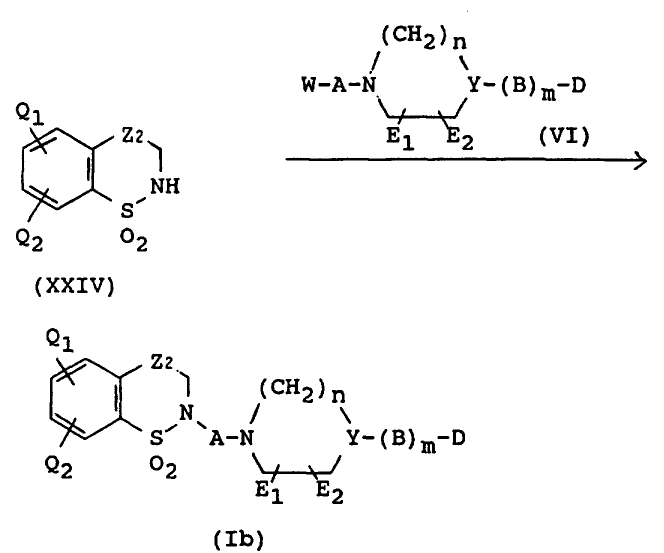 wherein A, B, D, E₁, E₂, Q₁, Q₂, W, Y, Z₂, m and n have the same meanings as defined above.

The conversion from the compound (XXIV) to the compound (Ib) can be conducted by causing the compound (VI) to act on the compound (XXIV) after treatment of the latter compound with an inorganic base or an organic base or in the presence of the base. Reaction conditions are similar to those employed in the conversion from the compound (XXIV) to the compound (XXV) in Process 1(a). In this case, it is also possible to add an alkali iodide such as potassium iodide or sodium iodide as needed. Incidentally, the compound (VI) can be synthesized by reacting the compound (V) with the compound (III) in a manner known *per se* in the art.

### Process 2 (Reference Process) :

Compounds (Ic) in which Z is represented by the following formula: 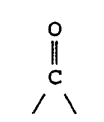 can be synthesized in any one of the following processes.

(a) The target compound can be obtained, in accordance with the following reaction scheme, by converting a compound (XV) or (XXV) to a compound (VIII) and then reacting the compound (VIII) with a compound represented by the formula (V): 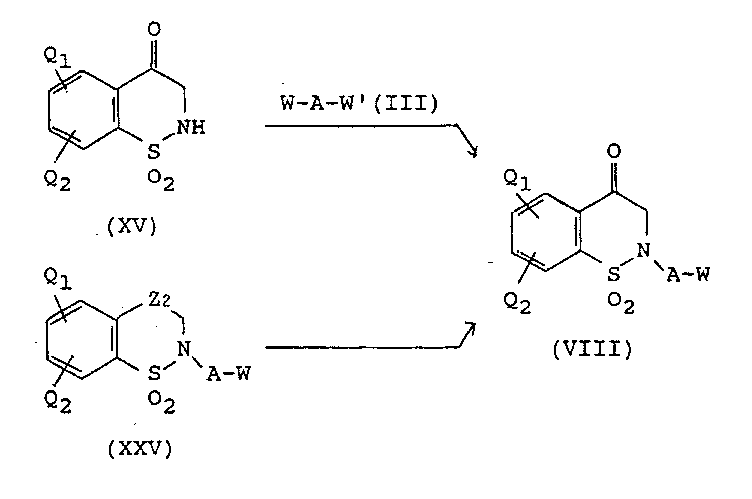 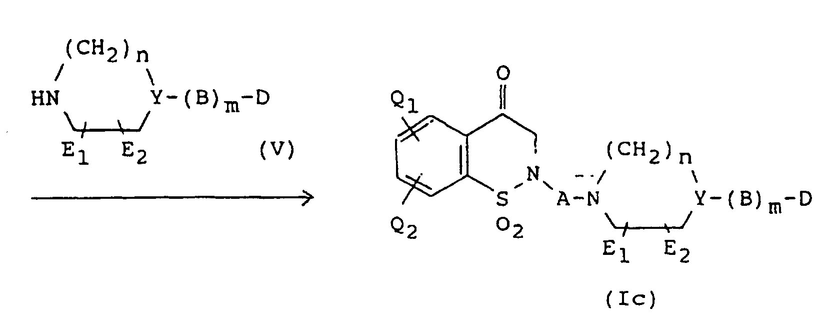 wherein A, B, D, E₁, E₂, Q₁, Q₂, W, W', Y, Z₂, m and n have the same meanings as defined above.

The conversion from the compound (XV) to the compound (VIII) can be effected under conditions similar to those employed upon conversion from the compound (XXIV) to the compound (XXV) shown in Process 1(a). Further, the conversion from the compound (XXV) to the compound (VIII) can be effected employing the process described by T.W. Greene in "Protective Groups in Organic Synthesis", John Wiley & Sons, Inc. and the like. For instance, the conversion to the target compound (VIII) can be conducted by acid treatment of the compound (XXV) when in Z₂, X₁ represents an oxygen atom or X₂ and X₃ both represent an oxygen atom, or by the treatment with mercury (II) chloride when X₁ represents a sulfur atom or X₂ and X₃ both represent a sulfur atom.

The conversion from the compound (VIII) to the compound (Ic) can be effected under conditions similar to those employed upon conversion from the compound (XXV) to the compound (Ib) shown in Process 1(a).

(b) The target compound can be obtained by the conversion of the group Z₂ of the compound (Ib) to a carbonyl group in accordance with the following reaction scheme. 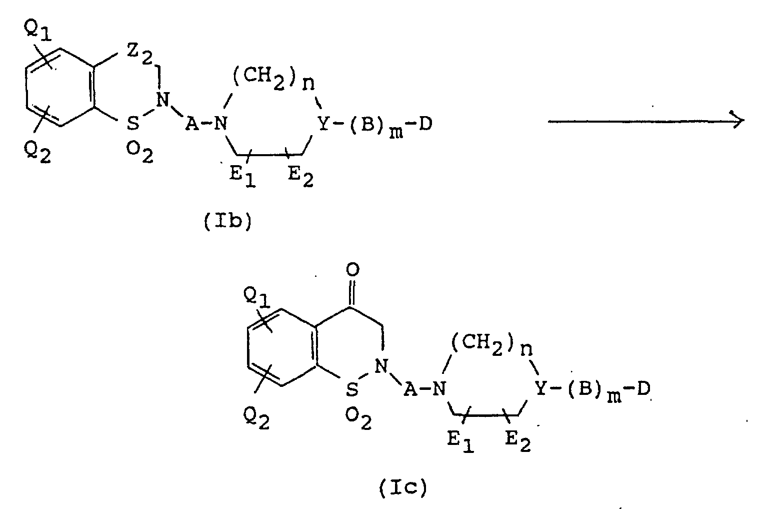 wherein A, B, D, E₁, E₂, E₁, Q₂, Y, Z₂, m and n have the same meanings as defined above.

The conversion from the compound (Ib) to the compound (Ic) can be effected under conditions similar to those employed in the conversion from the compound (XXV) to the compound (VIII) shown in Process 2(a).

### Process 3:

Among the substituted benzothiazine derivatives (I), each of compounds (Ih) and (If) in which Z is represented by the following formula: 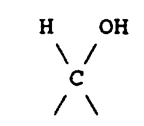 can be synthesized by any one of the following processes.

Incidentally, it is desired to select process (a) when there is a group reactive with a reducing agent in a nitrogen-containing compound (V).

(a) Each compound (Ih) can be obtained, in accordance with the following reaction scheme, by reducing the compound represented by the formula (VIII) to obtain the compound (X) and then causing the nitrogen-containing compound (V) to act on the resulting compound. 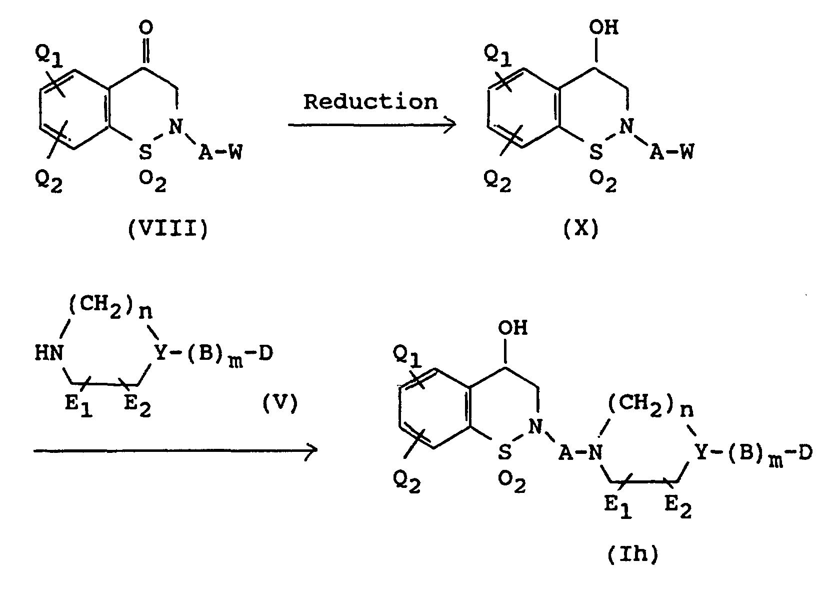 wherein A, B, D, E₁, E₂, Q₁, Q₂, W, Y, m and n have the same meanings as defined above.

The conversion from the compound (VIII) to the compound (X) can be effected by treating the compound represented by the formula (VIII) with a reducing agent such as sodium borohydride, potassium borohydride or sodium cyanoborohydride in a conventionally-employed solvent at -78°C to reflux temperature, preferably -20°C to room temperature.

Further, the conversion from the compound (X) to the compound (Ih) can be effected under conditions similar to those employed in the conversion from the compound (XXV) to the compound (Ib) shown in Process 1(a).

(b) Each compound (If) can be obtained by reducing the compound (Ic) in accordance with the following reaction scheme: 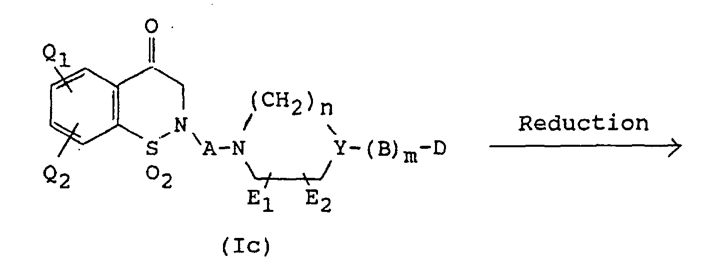 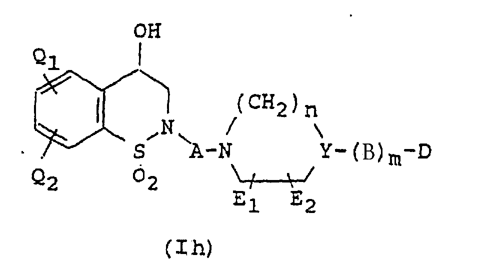 wherein A, B, D, E₁, E₂, Q₁, Q₂, Y, m and n have the same meanings as defined above.

The conversion from the compound (Ic) to the compound (If) can be effected under conditions similar to those employed in the conversion from the compound (VIII) to the compound (X) shown in Process 3 (a).

The compounds (I) of the present invention obtained according to the above-described processes can each be reacted with one of various acids to convert the compound to its salt. The salt can be purified by a method such as recrystallization or column chromatography.

Exemplary acids usable to convert the substituted benzothiazine derivatives (I) to their salts include inorganic acids such as hydrochloric acid, nitric acid, sulfuric acid, phosphoric acid and hydrobromic acid; and organic acids such as maleic acid, fumaric acid, tartaric acid, lactic acid, citric acid, acetic acid, methanesulfonic acid, p-toluenesulfonic acid, adipic acid, palmitic acid and tannic acid.

As will be demonstrated later by tests, the substituted benzothiazine derivatives (I) and their salts according to the present invention, which can be obtained as described above, have a strong serotonin-2 blocking action and in addition, they have excellent selectivity to α₁ blocking action. Further, as a result of a toxicity test, they have been found to feature high safety. The compounds according to the present invention can therefore be used as therapeutics for circulatory diseases such as ischemic heart diseases, cerebrovascular disturbances and peripheral circulatory disturbances.

When the substituted benzothiazine derivative (I) according to this invention are used as drugs, they can be administered in an effective dose as they are. As an alternative, they can also be formulated into various preparation forms by known methods and then administered.

Exemplary preparation forms as drugs include orally administrable preparation forms such as tablets, powders, granules, capsules and syrups as well as parenterally administrable preparation forms such as injections and suppositories. Whichever preparation form is used, a known liquid or solid extender or carrier usable for the formulation of the preparation form can be employed.

Examples of such extender or carrier include polyvinylpyrrolidone, arabic gum, gelatin, sorbit, cyclodextrin, tragacanth gum, magnesium stearate, talc, polyethylene glycol, polyvinyl alcohol, silica, lactose, crystalline cellulose, sugar, starch, calcium phosphate, vegetable oil, carboxymethylcellulose, sodium laurylsulfate, water, ethanol, glycerin, mannitol, syrup, and the like.

When the compounds (I) according to the present invention are used as drugs, their dose varies depending on the administration purpose, the age, body weight and conditions of the patient to be administered, etc. In oral administration, the daily dose may generally be about 0.01-1,000 mg.

The present invention will next be described in further detail by the following examples and tests. It is however borne in mind that the present invention is not limited to the following examples and tests.

### Example 1

### Synthesis of 2-acetonyl-4-methoxy-1,2-benzisothiazol-3(2H)-one 1,1-dioxide (Compound No. 1)

2.4 mℓ of 28% sodium methoxide solution in methanol (12 mmol) was added under ice cooling and stirring to a suspension of 2.34 g (11 mmol) of 4-methoxysaccharin in 20 mℓ of methanol, followed by stirring at 0°C for 1 hour. The reaction mixture was concentrated under reduced pressure, and the residue was washed with ethyl ether and dried, whereby the sodium salt of 4-methoxysaccharin was obtained in the form of powder.

A solution of the thus-obtained sodium salt and 1.22 g (13.2 mmol) of chloroacetone in 20 mℓ of DMF was stirred at 90-95°C for 5 hours. The reaction mixture was concentrated under reduced pressure. A 3:1 mixed solvent of ethyl acetate and dichloromethane was added to the residue. The resulting solution was washed successively with water, a saturated aqueous solution of sodium hydrogencarbonate and a saturated aqueous solution of sodium chloride, dried over anhydrous sodium sulfate, and then concentrated under reduced pressure. The residue was purified by chromatography on a silica gel column (eluent: methylene chloride:ethyl acetate = 50:1), whereby 2.78 g of the title compound were obtained (yield: 94%). Although that compound was sufficiently pure, it can be recrystallized from ethyl acetate-hexane as needed.

### Example 2

### Synthesis of 2-acetonyl-4-chloro-1,2-benzisothiazol-3(2H)-one 1,1-dioxide (Compound No. 2)

4.4 mℓ of 28% sodium methoxide solution in methanol (22 mmol) was added under ice cooling and stirring to a suspension of 4.36 g (20 mmol) of 4-chlorosaccharin in 40 mℓ of methanol, followed by stirring at 0°C for 30 minutes. The reaction mixture was treated as in Example 1, whereby the sodium salt of 4-chlorosaccharin was obtained in the form of powder.

A solution of the thus-obtained sodium salt and 2.21 g (24 mmol) of chloroacetone in 40 mℓ of DMF was stirred at 90-95°C for 15 hours. Post-treatments were conducted as in Example 1. The residue was purified by chromatography on a silica gel column (eluent: methylene chloride:ethyl acetate = 30:1), whereby 4.70 g of the title compound were obtained (yield: 86%). Although that compound was sufficiently pure, it can be recrystallized from ethyl acetate-hexane as needed.

### Example 3

### Synthesis of 2-acetonyl-5-methoxy-1,2-benzisothiazol-3(2H)-one 1,1-dioxide (Compound No. 3)

3.2 mℓ of 28% sodium methoxide solution in methanol (16 mmol) was added under ice cooling and stirring to a suspension of 3.10 g (14.5 mmol) of 5-methoxysaccharin in 30 mℓ of methanol, followed by stirring at 0°C for 15 minutes. The reaction mixture was treated as in Example 1, whereby the sodium salt of 5-methoxysaccharin was obtained in the form of powder.

A solution of the thus-obtained sodium salt and 2.23 g (24.2 mmol) of chloroacetone in 30 mℓ of DMF was stirred at 90-95°C for 5 hours. The reaction mixture was concentrated under reduced pressure. A 1:4 mixed solvent of ethyl acetate and dichloromethane was added to the residue. The resulting solution was washed successively with water, a saturated aqueous solution of sodium hydrogencarbonate and a saturated aqueous solution of sodium chloride, dried over anhydrous sodium sulfate, and then concentrated under reduced pressure. The residue was purified by chromatography on a silica gel column (eluent: methylene chloride:ethyl acetate = 50:1), whereby 3.85 g of the title compound were obtained (yield: 98%). Although that compound was sufficiently pure, it can be recrystallized from ethyl acetate-hexane as needed.

### Example 4

### Synthesis of 2-acetonyl-5-chloro-1,2-benzisothiazol-3(2H)-one 1,1-dioxide (Compound No. 4)

1.76 mℓ of 28% sodium methoxide solution in methanol (8.8 mmol) was added under ice cooling and stirring to a solution of 1.74 g (8 mmol) of 5-chlorosaccharin in 80 mℓ of methanol, followed by stirring at room temperature for 20 minutes. The reaction mixture was treated as in Example 1, whereby the sodium salt of 5-chlorosaccharin was obtained in the form of powder.

A solution of the thus-obtained sodium salt and 669 µℓ (8.4 mmol) of chloroacetone in 7 mℓ of DMF was stirred at 100°C for 15 hours. The reaction mixture was concentrated under reduced pressure. To the residue was added water, and extracted twice with chloroform. Organic layers were washed with a saturated aqueous solution of sodium chloride, dried over anhydrous sodium sulfate, and then concentrated under reduced pressure. The residue was purified by chromatography on a silica gel column (eluent: chloroform), whereby 1.38 g of the title compound were obtained (yield: 63%). Although that compound was sufficiently pure, it can be recrystallized from methanol as needed.

### Example 5

### Synthesis of 2-acetonyl-6-methoxy-1,2-benzisothiazol-3(2H)-one 1,1-dioxide (Compound No. 5)

2 mℓ of 28% sodium methoxide solution in methanol (10 mmol) was added under ice cooling and stirring to a suspension of 1.9 g (9 mmol) of 6-methoxysaccharin in 18 mℓ of methanol, followed by stirring at 0°C for 15 minutes. The reaction mixture was treated as in Example 1, whereby the sodium salt of 6-methoxysaccharin was obtained in the form of powder.

A solution of the thus-obtained sodium salt and 1.39 g (15 mmol) of chloroacetone in 18 mℓ of DMF was stirred at 90-95°C for 20 hours. Post-treatments and purification were conducted as in Example 3, whereby 2.26 g of the title compound were obtained (yield: 93%). Although that compound was sufficiently pure, it can be recrystallized from ethyl acetate-hexane as needed.

### Example 6

### Synthesis of 2-acetonyl-6-chloro-1,2-benzisothiazol-3(2H)-one 1,1-dioxide (Compound No. 6)

6.6 mℓ of 28% sodium methoxide solution in methanol (33 mmol) was added under ice cooling and stirring to a suspension of 6.54 g (30 mmol) of 6-chlorosaccharin in 60 mℓ of methanol, followed by stirring at 0°C for 15 minutes. The reaction mixture was treated as in Example 1, whereby the sodium salt of 6-chlorosaccharin was obtained in the form of powder.

A solution of the thus-obtained sodium salt and 3.30 g (36 mmol) of chloroacetone in 60 mℓ of DMF was stirred at 90-95°C for 12 hours. Post-treatments and purification were conducted as in Example 3, whereby 7.37 g of the title compound were obtained (yield: 89%). Although that compound was sufficiently pure, it can be recrystallized from ethyl acetate-hexane as needed.

### Example 7

### Synthesis of 3-acetyl-5-methoxy-2H-1,2-benzothiazin-4(3H)-one 1,1-dioxide (Compound No. 7)

Compound No. 1 (2.42 g, 9 mmol) was added at 45°C to a solution of 414 mg (18 mmol) of sodium in 9 mℓ of ethanol, followed by stirring for 10 minutes. The reaction mixture was ice-cooled, to which 11.3 mℓ (22.6 mmol) of 2 N hydrochloric acid were added. Precipitated crystals were collected by filtration, washed with water and then dried, whereby 1.55 g of the title compound were obtained (yield: 64%). Although that compound was sufficiently pure, it can be recrystallized from ethyl acetate-hexane as needed.

### Example 8

### Synthesis of 3-acetyl-5-chloro-2H-1,2-benzothiazin-4(3H)-one 1,1-dioxide (Compound No. 8)

Compound No. 2 (4.10 g, 15 mmol) was added at 50°C to a solution of 690 mg (30 mmol) of sodium in 15 mℓ of ethanol, followed by stirring for 10 minutes. The reaction mixture was ice-cooled, to which 18.7 mℓ (37.4 mmol) of 2 N hydrochloric acid were added. Precipitated crystals were collected by filtration, washed with water and then dried, whereby 3.00 g of the title compound were obtained (yield: 73%). Although that compound was sufficiently pure, it can be recrystallized from ethyl acetate-hexane as needed.

### Example 9

### Synthesis of 3-acetyl-6-methoxy-2H-1,2-benzothiazin-4(3H)-one 1,1-dioxide (Compound No. 9)

Compound No. 3 (3.50 g, 13 mmol) was added at 50°C to a solution of 600 mg (26 mmol) of sodium in 13 mℓ of ethanol, followed by stirring for 10 minutes. The reaction mixture was ice-cooled, to which 15 mℓ (30 mmol) of 2 N hydrochloric acid were added. Precipitated crystals were collected by filtration, washed with water and then dried, whereby 2.90 g of the title compound were obtained (yield: 83%). Although that compound was sufficiently pure, it can be recrystallized from ethyl acetate-hexane as needed.

### Example 10

### Synthesis of 3-acetyl-6-chloro-2H-1,2-benzothiazin-4(3H)-one 1,1-dioxide (Compound No. 10)

Compound No. 4 (1.26 g, 4.6 mmol) was added at 40°C to a solution of 212 mg (9.2 mmol) of sodium in 4.1 mℓ of ethanol, followed by stirring at 50-55°C for 5 minutes. The reaction mixture was ice-cooled, to which 5.75 mℓ (11.5 mmol) of 2 N hydrochloric acid were added. Precipitated crystals were collected by filtration, washed with 50% water-ethanol and then dried, whereby 1.15 g of the title compound were obtained (yield: 91%). Although that compound was sufficiently pure, it can be recrystallized from ethanol-water as needed.

### Example 11

### Synthesis of 3-acetyl-7-methoxy-2H-1,2-benzothiazin-4(3H)-one 1,1-dioxide (Compound No. 11)

Compound No. 5 (2.15 g, 8 mmol) was added at 45°C to a solution of 368 mg (16 mmol) of sodium in 7.2 mℓ of ethanol, followed by stirring for 10 minutes. The reaction mixture was ice-cooled, to which 10 mℓ (20 mmol) of 2 N hydrochloric acid were added. Precipitated crystals were collected by filtration, washed with water and then dried, whereby 1.70 g of the title compound were obtained (yield: 81%). Although that compound was sufficiently pure, it can be recrystallized from ethyl acetate-hexane as needed.

### Example 12

### Synthesis of 3-acetyl-7-chloro-2H-1,2-benzothiazin-4(3H)-one 1,1-dioxide (Compound No. 12)

Compound No. 6 (6.84 g, 25 mmol) was added at 45°C to a solution of 1.15 g (50 mmol) of sodium in 22.5 mℓ of ethanol, followed by stirring for 10 minutes. The mixture was ice-cooled, to which 32 mℓ (64 mmol) of 2 N hydrochloric acid were added. Precipitated crystals were collected by filtration, washed with water and then dried, whereby 6.18 g of the title compound were obtained (yield: 90%). Although that compound was sufficiently pure, it can be recrystallized from ethanol-water as needed.

### Example 13

### Synthesis of 5-methoxy-3,4-dihydro-2H-1,2-benzothiazin-4-one 1,1-dioxide ethylene acetal (Compound No. 13)

A solution of 935 mg (3.4 mmol) of Compound No. 7, 1.05 g (17 mmol) of ethylene glycol and 65 mg (0.34 mmol) of p-toluenesulfonic acid monohydrate in 30 mℓ of benzene was refluxed in a vessel equipped with a Dean & Stark water separator. Seventy (70) hours later, 1.05 g (17 mmol) of ethylene glycol were added, followed by further reflux for 24 hours. The solvent was concentrated to 15 mℓ under reduced pressure and crystals were collected by filtration. They were recrystallized from acetonitrile, whereby 590 mg of the title compound were obtained (yield: 64%).

### Example 14

### Synthesis of 5-chlbro-3,4-dihydro-2H-1,2-benzothiazin-4-one 1,1-dioxide ethylene acetal (Compound No. 14)

A solution of 2.73 g (10 mmol) of Compound No. 8, 3.10 g (50 mmol) of ethylene glycol and 190 mg (1 mmol) of p-toluenesulfonic acid monohydrate in 100 mℓ of benzene was refluxed in a vessel equipped with a Dean & Stark water separator. Eighty-four (84) hours later, 3.10 g (50 mmol) of ethylene glycol and 190 mg (1 mmol) of p-toluenesulfonic acid monohydrate were added, followed by further reflux for 84 hours. The reaction mixture was concentrated under reduced pressure, ethyl acetate was added to the residue, and crystals were collected by filtration. They were recrystallized from acetonitrile, whereby 1.00 g of the title compound was obtained (yield: 36%).

### Example 15

### Synthesis of 6-methoxy-3,4-dihydro-2H-1,2-benzothiazin-4-one 1,1-dioxide ethylene acetal (Compound No. 15)

A solution of 2.70 g (10 mmol) of Compound No. 9, 3.10 g (50 mmol) of ethylene glycol and 190 mg (1 mmol) of p-toluenesulfonic acid monohydrate in 30 mℓ of benzene was refluxed for 140 hours in a vessel equipped with a Dean & Stark water separator. Ethyl acetate was added to the reaction mixture. The resultant mixture was washed successively with a saturated aqueous solution of sodium hydrogencarbonate, water and a saturated aqueous solution of sodium chloride, dried over anhydrous sodium sulfate, and then concentrated under reduced pressure. The residue was purified by chromatography on a silica gel column (eluent: ethyl acetate:hexane = 1:2) and then recrystallized from acetonitrile, whereby 1.00 g of the title compound was obtained (yield: 37%).

### Example 16

### Synthesis of 6-chloro-3,4-dihydro-2H-1,2-benzothiazin-4-one 1,1-dioxide ethylene acetal (Compound No. 16)

A solution of 4.70 g (17 mmol) of Compound No. 10, 5.30 g (85 mmol) of ethylene glycol and 323 mg (1.7 mmol) of p-toluenesulfonic acid monohydrate in 50 mℓ of benzene was refluxed in a vessel equipped with a Dean & Stark water separator. One hundred and forty-four (144) hours later, 930 mg (15 mmol) of ethylene glycol were added, followed by further reflux for 20 hours. Post-treatments were conducted as in Example 15. The residue was recrystallized from ethyl acetate-hexane, whereby 2.07 g of the title compound were obtained (yield: 44%).

### Example 17

### Synthesis of 7-methoxy-3,4-dihydro-2H-1,2-benzothiazin-4-one 1,1-dioxide ethylene acetal (Compound No. 17)

A solution of 1.70 g (6.3 mmol) of Compound No. 11, 2.00 g (32 mmol) of ethylene glycol and 60 mg (0.31 mmol) of p-toluenesulfonic acid monohydrate in 15 mℓ of benzene was refluxed in a vessel equipped with a Dean & Stark water separator. One hundred and forty-four (144) hours later, 465 mg (7.5 mmol) of ethylene glycol were added, followed by further reflux for 20 hours. Post-treatments were conducted as in Example 15. The residue was purified by chromatography on a silica gel column (eluent: methylene chloride: ethyl acetate = 40:1), whereby 314 mg of the title compound were obtained (yield: 18%). Although that compound was sufficiently pure, it can be recrystallized from ethyl acetate-hexane as needed.

### Example 18

### Synthesis of 7-chloro-3,4-dihydro-2H-1,2-benzothiazin-4-one 1,1-dioxide ethylene acetal (Compound No. 18)

A solution of 5.48 g (20 mmol) of Compound No. 12, 6.2 g (100 mmol) of ethylene glycol and 125 mg (0.67 mmol) of p-toluenesulfonic acid monohydrate in 50 mℓ of benzene was refluxed in a vessel equipped with a Dean & Stark water separator. Two hundred and forty (240) hours later, 2.25 g (37.5 mmol) of ethylene glycol were added, followed by further reflux for 20 hours. Post-treatments and purification were conducted as in Example 17, whereby 1.77 g of the title compound were obtained (yield: 32%). Although that compound was sufficiently pure, it can be recrystallized from ethyl acetate-hexane as needed.

### Example 19

### Synthesis of 5-chloro-3,4-dihydro-2H-1,2-benzothiazin-4-one 1,1-dioxide (Compound No. 19)

3 N hydrochloric acid (10 mℓ, 30 mmol) was added to a suspension of 414 mg (1.5 mmol) of Compound No. 14 in 10 mℓ of methanol, followed by reflux for 70 minutes. The reaction mixture was concentrated under reduced pressure and precipitated crystals were collected by filtration. Those crystals were dissolved in ethyl acetate. The resulting solution was washed with a saturated aqueous solution of sodium chloride, dried over anhydrous sodium sulfate and then concentrated under reduced pressure, whereby 362 mg of the title compound were obtained (yield: 94%). Although that compound was sufficiently pure, it can be recrystallized from ethanol as needed.

### Example 20

### Synthesis of 5-chloro-3,4-dihydro-2H-1,2-benzothiazine-4-spiro-2'-(1',3'-dithiolane) 1,1-dioxide (Compound No. 20)

Boron trifluoride-ether complex (62 µℓ, 0.5 mmol) was added under ice cooling and stirring to a suspension of 232 mg (1 mmol) of Compound No. 19 and 252 µℓ (3 mmol) of 1,2-ethanedithiol in 5 mℓ of methylene chloride. The resultant mixture was stirred at 0°C for 70 minutes and then at room temperature. Twenty-four (24) hours later, 31 µℓ (0.25 mmol) of boron trifluoride-ether complex were added and seven (7) hours later, 63 µℓ (0.75 mmol) of 1,2-ethanedithiol and 31 µℓ (0.25 mmol) of boron trifluoride were added further, followed by stirring for 65 hours.

An aqueous solution of potassium carbonate was added to the reaction mixture, and the mixture so obtained was extracted twice with methylene chloride. Organic layers were washed with a saturated aqueous solution of sodium chloride, dried over anhydrous sodium sulfate, and then concentrated under reduced pressure. The residue was purified by chromatography on a silica gel column (eluent: methylene chloride: methanol = 30:1), whereby 223 mg of the title compound were obtained (yield: 72%). Although that compound was sufficiently pure, it can be recrystallized from chloroform-hexane as needed.

### Example 21

### Synthesis of 2-(2-chloroethyl)-5-methoxy-3,4-dihydro-2H-1,2-benzothiazin-4-one 1,1-dioxide ethylene acetal (Compound No. 21)

A solution of 190 mg (0.7 mmol) of Compound No. 13 in 5 mℓ of DMF was added under ice cooling and stirring to a suspension of 31 mg (0.77 mmol) of 60% sodium hydride in 5 mℓ of DMF. After the reaction mixture was stirred at 0°C for 1 hour and then at room temperature for 1 hour, the reaction mixture was ice-cooled. A solution of 201 mg (1.4 mmol) of 1-bromo-2-chloroethane in 5 mℓ of DMF was then added, followed by stirring at room temperature for 16 hours.

The reaction mixture was concentrated under reduced pressure and 50 mℓ of a 5% aqueous solution of citric acid were added to the residue. The resultant mixture was extracted three times with methylene chloride. Organic layers were washed successively with water and a saturated aqueous solution of sodium chloride, dried over anhydrous sodium sulfate, and then concentrated under reduced pressure. The residue was purified by chromatography on a silica gel column (eluent: ethyl acetate:hexane =1:1), whereby 213 mg of the title compound were obtained (yield: 91%). Although that compound was sufficiently pure, it can be recrystallized from ethyl acetate-hexane as needed.

### Example 22

### Synthesis of 2-(3-chloropropyl)-5-methoxy-3,4-dihydro-2H-1,2-benzothiazin-4-one 1,1-dioxide ethylene acetal (Compound No. 22)

A suspension of 813 mg (3 mmol) of Compound No. 13, 945 mg (6 mmol) of 1-bromo-3-chloropropane and 828 mg (6 mmol) of potassium carbonate in 15 mℓ of DMF was stirred at room temperature for 15 hours. The reaction mixture was filtered and the filtrate was concentrated under reduced pressure.

Ethyl acetate was added to the residue. The mixture so obtained was washed successively with a saturated aqueous solution of sodium hydrogencarbonate and a saturated solution of sodium chloride, dried over anhydrous magnesium sulfate, and then concentrated under reduced pressure. The residue was purified by chromatography on a silica gel column (eluent: methylene chloride:ethyl acetate = 50:1), whereby 1.03 g of the title compound were obtained (yield: 98%). Although that compound was sufficiently pure, it can be recrystallized from ethyl acetate-hexane as needed.

### Example 23

### Synthesis of 5-chloro-2-(3-chloropropyl)-3,4-dihydro-2H-1,2-benzothiazin-4-one 1,1-dioxide ethylene acetal (Compound No. 23)

A suspension of 551 mg (2 mmol) of Compound No. 14, 630 mg (4 mmol) of 1-bromo-3-chloropropane and 552 mg (4 mmol) of potassium carbonate in 10 mℓ of DMF was stirred at room temperature for 20 hours. Post-treatments and purification were conducted as in Example 22, whereby 698 mg of the title compound were obtained (yield: 99%). Although that compound was sufficiently pure, it can be recrystallized from ethyl acetate-hexane as needed.

### Example 24

### Synthesis of 2-(3-chloropropyl)-6-methoxy-3,4-dihydro-2H-1,2-benzothiazin-4-one 1,1-dioxide ethylene acetal (Compound No. 24)

A suspension of 542 mg (2 mmol) of Compound No. 15, 471 mg (3 mmol) of 1-bromo-3-chloropropane and 552 mg (4 mmol) of potassium carbonate in 20 mℓ of acetone was refluxed for 8 hours. The reaction mixture was filtered and the filtrate was concentrated under reduced pressure.

Ethyl acetate was added to the residue. The mixture so obtained was washed successively with a saturated aqueous solution of sodium hydrogencarbonate and a saturated solution of sodium chloride, dried over anhydrous magnesium sulfate, and then concentrated under reduced pressure. The residue was purified by chromatography on a silica gel column (eluent: ethyl acetate:hexane = 1:2), whereby 578 mg of the title compound were obtained (yield: 83%). Although that compound was sufficiently pure, it can be recrystallized from ethyl acetate-hexane as needed.

### Example 25

### Synthesis of 6-chloro-2-(3-chloropropyl)-3,4-dihydro-2H-1,2-benzothiazin-4-one 1,1-dioxide ethylene acetal (Compound No. 25)

A suspension of 550 mg (2 mmol) of Compound No. 16, 471 mg (3 mmol) of 1-bromo-3-chloropropane and 552 mg (4 mmol) of potassium carbonate in 20 mℓ of acetone was refluxed for 14 hours. Post-treatments and purification were conducted as in Example 24, whereby 735 mg of the title compound were obtained (yield: 99%). Although that compound was sufficiently pure, it can be recrystallized from ethyl acetate-hexane as needed.

### Example 26

Synthesis of 2-(3-chloropropyl)-7-methoxy-3,4-dihydro-2H-1,2-benzothiazin-4-one 1,1-dioxide ethylene acetal (Compound No. 26)

A suspension of 271 mg (1 mmol) of Compound No. 17, 236 mg (1.5 mmol) of 1-bromo-3-chloropropane and 276 mg (2 mmol) of potassium carbonate in 10 mℓ of acetone was refluxed for 10 hours. Post-treatments and purification were conducted as in Example 24, whereby 321 mg of the title compound were obtained (yield: 92%). Although that compound was sufficiently pure, it can be recrystallized from ethyl acetate-hexane as needed.

### Example 27

Synthesis of 7-chloro-2-(3-chloropropyl)-3,4-dihydro-2H-1,2-benzothiazin-4-one 1,1-dioxide ethylene acetal (Compound No. 27)

A suspension of 825 mg (3 mmol) of Compound No. 18, 707 mg (4.5 mmol) of 1-bromo-3-chloropropane and 828 mg (6 mmol) of potassium carbonate in 30 mℓ of acetone was refluxed for 7 hours. Post-treatments were conducted as in Example 24 and the residue was purified by chromatography on a silica gel column (eluent: ethyl acetate:hexane = 1:1), whereby 1.08 g of the title compound were obtained (yield: 99%). Although that compound was sufficiently pure, it can be recrystallized from ethyl acetate-hexane as needed.

### Example 28

Synthesis of 2-(3-chloropropyl)-5-methoxy-3,4-dihydro-2H-1,2-benzothiazin-4-one 1,1-dioxide (Compound No. 28)

4 N hydrochloric acid (4 mℓ, 16 mmol) was added to a solution of 430 mg (1.24 mmol) of Compound No. 22 in 4 mℓ of methanol, followed by reflux for 2 hours. The reaction mixture was concentrated under reduced pressure. The residue was added with ethyl acetate. The mixture so obtained was washed successively with a saturated aqueous solution of sodium hydrogencarbonate and a saturated solution of sodium chloride, dried over anhydrous magnesium sulfate, and then concentrated under reduced pressure. The residue was purified by chromatography on a silica gel column (eluent: methylene chloride:ethyl acetate = 30:1), whereby 392 mg of the title compound were obtained (yield: 99%).

### Example 29

### Synthesis of 2-(3-chloropropyl)-5-methoxy-3,4-dihydro-2H-1,2-benzothiazine-4-spiro-2'-(1',3'-dithiolane) 1,1-dioxide (Compound No. 29)

Boron trifluoride-ether complex (0.06 mℓ, 0.45 mmol) was added under ice cooling and stirring to a solution of 350 mg (1.15 mmol) of Compound No. 28 and 0.12 mℓ (1.4 mmol) of ethanedithiol in 4.5 mℓ of methylene chloride. The resultant mixture was stirred at 0°C for 1 hour and then at room temperature for 12 hours.

Ethyl acetate was added to the reaction mixture. The mixture so obtained was washed successively with a saturated aqueous solution of sodium hydrogencarbonate and a saturated solution of sodium chloride, dried over anhydrous magnesium sulfate, and then concentrated under reduced pressure. The residue was purified by chromatography on a silica gel column (eluent: ethyl acetate:hexane = 1:2) and then recrystallized from ethyl acetate-hexane, whereby 383 mg of the title compound were obtained (yield: 87%).

### Example 30

Synthesis of 2-[2-[4-(4-fluorophenyl)piperazin-1-yl]ethyl]-5-methoxy-3,4-dihydro-2H-1,2-benzothiazin-4-one 1,1-dioxide ethylene acetal (Compound No. 30)

A suspension of 100 mg (0.3 mmol) of Compound No. 21, 108 mg (0.6 mmol) of 1-(4-fluorophenyl)piperazine and 90 mg (0.6 mmol) of sodium iodide in 15 mℓ of acetonitrile was refluxed for 24 hours.

The reaction mixture was concentrated under reduced pressure and 50 mℓ of a half-saturated aqueous solution of potassium carbonate were added to the residue. The mixture so obtained was extracted three times with ethyl acetate. Organic layers were washed successively with water and a saturated aqueous solution of sodium chloride, dried over anhydrous sodium sulfate, and then concentrated under reduced pressure. The residue was purified by chromatography on a silica gel column (eluent: ethyl acetate:hexane = 3:1), whereby 106 mg of the title compound were obtained (yield: 74%). Although that compound was sufficiently pure, it can be recrystallized from ethyl acetate-hexane as needed.

### Example 31

Synthesis of 2-[3-[4-(4-fluorophenyl)piperazin-1-yl]propyl]-5-methoxy-3,4-dihydro-2H-1,2-benzothiazin-4-one 1,1-dioxide ethylene acetal (Compound No. 31)

A suspension of 173 mg (0.5 mmol) of Compound No. 22, 135 mg (0.75 mmol) of 1-(4-fluorophenyl)piperazine, 84 mg (1 mmol) of sodium hydrogencarbonate and 150 mg (1 mmol) of sodium iodide in 10 mℓ of acetonitrile was refluxed for 18 hours.

The reaction mixture was concentrated under reduced pressure and a saturated aqueous solution of sodium hydrogencarbonate was added to the residue. The mixture so obtained was extracted twice with methylene chloride. Organic layers were dried over anhydrous magnesium sulfate and then concentrated under reduced pressure. The residue was purified by chromatography on a silica gel column (eluent: methylene chloride: methanol = 30:1), whereby 243 mg of the title compound were obtained (yield: 98%). Although that compound was sufficiently pure, it can be recrystallized from ethyl acetate-hexane as needed.

### Example 32

Synthesis of 2-[3-[4-(4-hydroxyphenyl)piperazin-1-yl]propyl]-5-methoxy-3,4-dihydro-2H-1,2-benzothiazin-4-one 1,1-dioxide ethylene acetal (Compound No. 32)

A suspension of 173 mg (0.5 mmol) of Compound No. 22, 194 mg (0.75 mmol) of 1-(4-hydroxyphenyl)piperazine hydrobromide, 168 mg (2 mmol) of sodium hydrogencarbonate and 150 mg (1 mmol) of sodium iodide in 10 mℓ of acetonitrile was refluxed for 20 hours.

Post-treatments were conducted as in Example 31 and the residue was purified by chromatography on a silica gel column (eluent: methylene chloride: methanol . = 20:1), whereby 191 mg of the title compound were obtained (yield: 78%). Although that compound was sufficiently pure, it can be recrystallized from 2-propanol as needed.

### Example 33

### Synthesis of 2-[3-[4-(4-fluorobenzoyl)piperidino]propyl]-5-methoxy-3,4-dihydro-2H-1,2-benzothiazin-4-one 1,1-dioxide ethylene acetal (Compound No. 33)

A suspension of 173 mg (0.5 mmol) of Compound No. 22, 183 mg (0.75 mmol) of 4-(4-fluorobenzoyl)piperidine hydrochloride, 168 mg (2 mmol) of sodium hydrogencarbonate and 150 mg (1 mmol) of sodium iodide in 10 mℓ of acetonitrile was refluxed for 18 hours. Post-treatments and purification were conducted as in Example 31, whereby 247 mg of the title compound were obtained (yield: 95%).

### Example 34

### Synthesis of 5-chloro-2-[3-[4-(4-fluorophenyl)piperazin-1-yl]propyl]-3,4-dihydro-2H-1,2-benzothiazin-4-one 1,1-dioxide ethylene acetal (Compound No. 34)

A suspension of 141 mg (0.4 mmol) of Compound No. 23, 108 mg (0.6 mmol) of 1-(4-fluorophenyl)piperazine, 67 mg (0.8 mmol) of sodium hydrogencarbonate and 120 mg (0.8 mmol) of sodium iodide in 8 mℓ of acetonitrile was refluxed for 16 hours. Post-treatments and purification were conducted as in Example 31, whereby 196 mg of the title compound were obtained (yield: 98%).

### Example 35

### Synthesis of 5-chloro-2-[3-[4-(4-hydroxyphenyl)piperazin-1-yl]propyl]-3,4-dihydro-2H-1,2-benzothiazin-4-one 1,1-dioxide ethylene acetal (Compound No. 35)

A suspension of 141 mg (0.4 mmol) of Compound No. 23, 155 mg (0.6 mmol) of 1-(4-hydroxyphenyl)piperazine hydrobromide, 134 mg (1.6 mmol) of sodium hydrogencarbonate and 120 mg (0.8 mmol) of sodium iodide in 8 mℓ of acetonitrile was refluxed for 17 hours. Post-treatments and purification were conducted as in Example 32, whereby 181 mg of the title compound were obtained (yield: 93%).

### Example 36

### Synthesis of 2-[3-[4-(4-fluorophenyl)piperazin-1-yl]propyl]-6-methoxy-3,4-dihydro-2H-1,2-benzothiazin-4-one 1,1-dioxide ethylene acetal (Compound No. 36)

A suspension of 173 mg (0.5 mmol) of Compound No. 24, 135 mg (0.75 mmol) of 1-(4-fluorophenyl)piperazine, 84 mg (1 mmol) of sodium hydrogencarbonate and 150 mg (1 mmol) of sodium iodide in 10 mℓ of acetonitrile was refluxed for 15 hours. Post-treatments and purification were conducted as in Example 32, whereby 229 mg of the title compound were obtained (yield: 93%). Although that compound was sufficiently pure, it can be recrystallized from ethyl acetate-hexane as needed.

### Example 37

### Synthesis of 2-[3-[4-(4-fluorobenzoyl)piperidino]propyl]-6-methoxy-3,4-dihydro-2H-1,2-benzothiazin-4-one 1,1-dioxide ethylene acetal (Compound No. 37)

A suspension of 173 mg (0.5 mmol) of Compound No. 24, 182 mg (0.75 mmol) of 4-(4-fluorobenzoyl)piperidine hydrochloride, 168 mg (2 mmol) of sodium hydrogencarbonate and 150 mg (1 mmol) of sodium iodide in 10 mℓ of acetonitrile was refluxed for 15 hours. Post-treatments were conducted as in Example 31 and the residue was purified by chromatography on a silica gel column (eluent: methylene chloride: methanol = 15:1), whereby 241 mg of the title compound were obtained (yield: 93%). Although that compound was sufficiently pure, it can be recrystallized from ethyl acetate-hexane as needed.

### Example 38

### Synthesis of 6-chloro-2-[3-[4-(4-fluorophenyl)piperazin-1-yl]propyl]-3,4-dihydro-2H-1,2-benzothiazin-4-one 1,1-dioxide ethylene acetal (Compound No. 38)

A suspension of 176 mg (0.5 mmol) of Compound No. 25, 135 mg (0.75 mmol) of 1-(4-fluorophenyl)piperazine, 84 mg (1 mmol) of sodium hydrogencarbonate and 150 mg (1 mmol) of sodium iodide in 10 mℓ of acetonitrile was refluxed for 15 hours. Post-treatments and purification were conducted as in Example 32, whereby 206 mg of the title compound were obtained (yield: 83%).

### Example 39

### Synthesis of 6-chloro-2-[3-[4-(4-fluorobenzoyl)piperidino]propyl]-3,4-dihydro-2H-1,2-benzothiazin-4-one 1,1-dioxide ethylene acetal (Compound No. 39)

A suspension of 176 mg (0.5 mmol) of Compound No. 25, 182 mg (0.75 mmol) of 4-(4-fluorobenzoyl)piperidine hydrochloride, 168 mg (2 mmol) of sodium hydrogencarbonate and 150 mg (1 mmol) of sodium iodide in 10 mℓ of acetonitrile was refluxed for 15 hours. Post-treatments and purification were conducted as in Example 32, whereby 211 mg of the title compound were obtained (yield: 80%). Although that compound was sufficiently pure, it can be recrystallized from ethyl acetate-hexane as needed.

### Example 40

### Synthesis of 2-[3-[4-(4-fluorophenyl)piperazin-1-yl]propyl]-7-methoxy-3,4-dihydro-2H-1,2-benzothiazin-4-one 1,1-dioxide ethylene acetal (Compound No. 40)

A suspension of 104 mg (0.3 mmol) of Compound No. 26, 81 mg (0.45 mmol) of 1-(4-fluorophenyl)piperazine, 51 mg (0.6 mmol) of sodium hydrogencarbonate and 90 mg (0.6 mmol) of sodium iodide in 6 mℓ of acetonitrile was refluxed for 18 hours. Post-treatments and purification were conducted as in Example 31, whereby 138 mg of the title compound were obtained (yield: 94%). Although that compound was sufficiently pure, it can be recrystallized from ethyl acetate-hexane as needed.

### Example 41

### Synthesis of 2-[3-[4-(4-fluorobenzoyl)piperidino]propyl]-7-methoxy-3,4-dihydro-2H-1,2-benzothiazin-4-one 1,1-dioxide ethylene acetal (Compound No. 41)

A suspension of 104 mg (0.3 mmol) of Compound No. 26, 109 mg (0.45 mmol) of 4-(4-fluorobenzoyl)piperidine hydrochloride, 101 mg (1.2 mmol) of sodium hydrogencarbonate and 90 mg (0.6 mmol) of sodium iodide in 6 mℓ of acetonitrile was refluxed for 18 hours. Post-treatments and purification were conducted as in Example 32, whereby 140 mg of the title compound were obtained (yield: 90%). Although that compound was sufficiently pure, it can be recrystallized from ethyl acetate-hexane as needed.

### Example 42

### Synthesis of 7-chloro-2-[3-[4-(4-fluorophenyl)piperazin-1-yl]propyl]-3,4-dihydro-2H-1,2-benzothiazin-4-one 1,1-dioxide ethylene acetal (Compound No. 42)

A suspension of 160 mg (0.45 mmol) of Compound No. 27, 135 mg (0.75 mmol) of 1-(4-fluorophenyl)piperazine, 84 mg (2 mmol) of sodium hydrogencarbonate and 150 mg (1 mmol) of sodium iodide in 10 mℓ of acetonitrile was refluxed for 15 hours. Post-treatments and purification were conducted as in Example 31, whereby 194 mg of the title compound were obtained (yield: 78%). Although that compound was sufficiently pure, it can be recrystallized from ethyl acetate-hexane as needed.

### Example 43

### Synthesis of 7-chloro-2-[3-[4-(4-fluorobenzoyl)piperidino]propyl]-3,4-dihydro-2H-1,2-benzothiazin-4-one 1,1-dioxide ethylene acetal (Compound No. 43)

A suspension of 160 mg (0.45 mmol) of Compound No. 27, 182 mg (0.75 mmol) of 4-(4-fluorobenzoyl)piperidine hydrochloride, 168 mg (2 mmol) of sodium hydrogencarbonate and 150 mg (1 mmol) of sodium iodide in 10 mℓ of acetonitrile was refluxed for 15 hours. Post-treatments and purification were conducted as in Example 32, whereby 179 mg of the title compound were obtained (yield: 68%). Although that compound was sufficiently pure, it can be recrystallized from ethyl acetate-hexane as needed.

### Example 44

### Synthesis of 2-[3-[4-(4-fluorophenyl)piperazin-1-yl]propyl]-5-methoxy-3,4-dihydro-2H-1,2-benzothiazine-4-spiro-2'-(1',3'-dithiolane) 1,1-dioxide (Compound No. 44)

A suspension of 190 mg (0.5 mmol) of Compound No. 29, 135 mg (0.75 mmol) of 1-(4-fluorophenyl)piperazine, 84 mg (1 mmol) of sodium hydrogencarbonate and 150 mg (1 mmol) of sodium iodide in 10 mℓ of acetonitrile was refluxed for 18 hours. Post-treatments and purification were conducted as in Example 31, whereby 223 mg of the title compound were obtained (yield: 90%). Although that compound was sufficiently pure, it can be recrystallized from methanol as needed.

### Example 45

### Synthesis of 5-chloro-2-[3-[4-(4-fluorophenyl)piperazin-1-yl]propyl]-3,4-dihydro-2H-1,2-benzothiazine-4-spiro-2'-(1',3'-dithiolane) 1,1-dioxide (Compound No. 45)

A solution of 154 mg (0.5 mmol) of Compound No. 20 in 1.5 mℓ of DMF was added under ice cooling and stirring to a suspension of 22 mg (0.55 mmol) of 60%-sodium hydride in 3 mℓ of DMF, followed by stirring at 0°C for 30 minutes and then at room temperature for 30 minutes. The reaction mixture was then cooled down to 0°C, to which a solution of 193 mg (0.75 mmol) of 1-(3-chloropropyl)-4-(4-fluorophenyl)piperazine in 1.5 mℓ of DMF was added. The resulting mixture was stirred at room temperature for 24 hours.

A 3:1 (by volume) mixed solvent of ethyl acetate and benzene was added to the reaction mixture. An organic layer was washed successively with a half-saturated aqueous solution of sodium hydrogencarbonate, water and a saturated solution of sodium chloride, dried over anhydrous sodium sulfate, and then concentrated under reduced pressure. The residue was purified by chromatography on a silica gel column (eluent: ethyl acetate:hexane = 1:2 → 1:1 → 2:1), whereby 102 mg of the title compound were obtained (yield: 39%). Although that compound was sufficiently pure, it can be recrystallized from ethyl acetate-hexane as needed.

The structural formulas and physical properties of the compounds obtained in the above examples are summarized in Table 1 to Table 12.

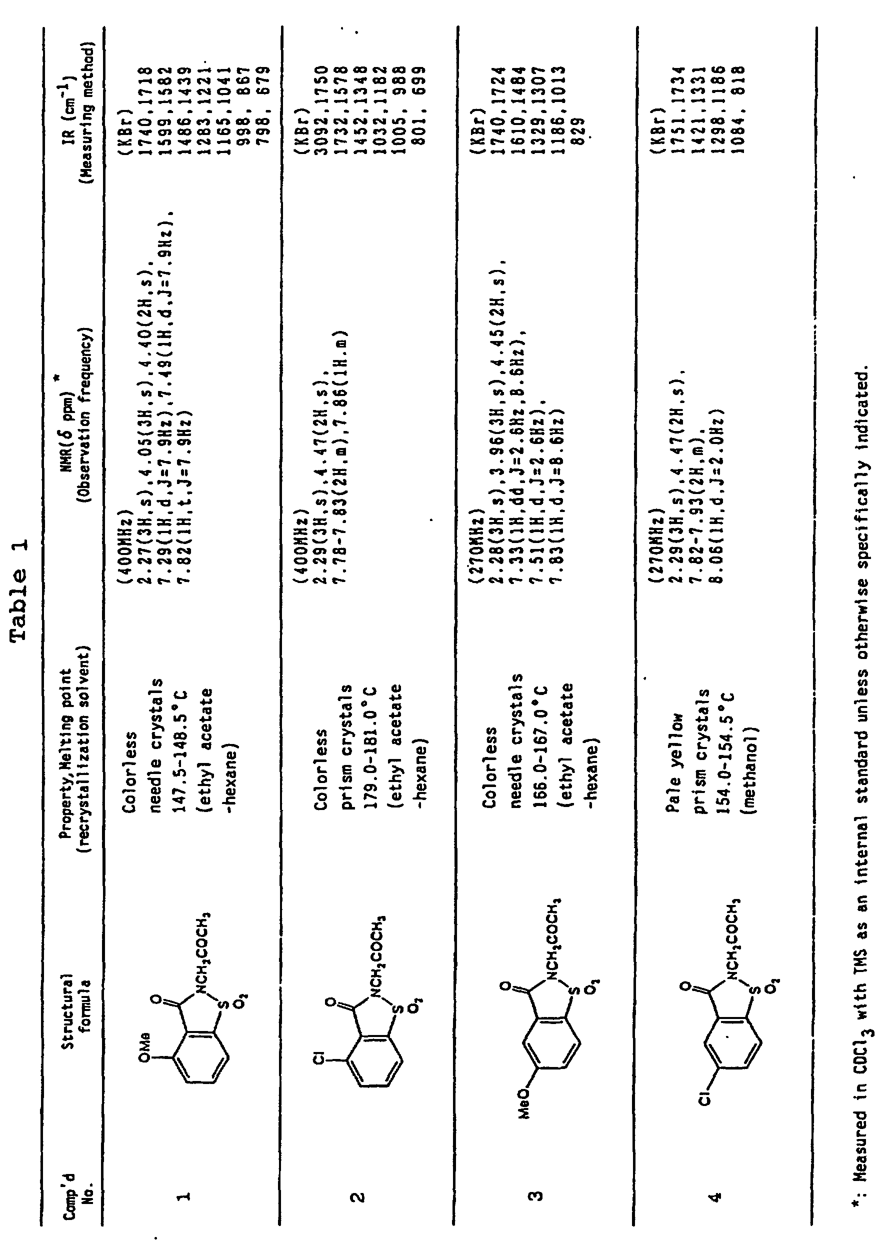

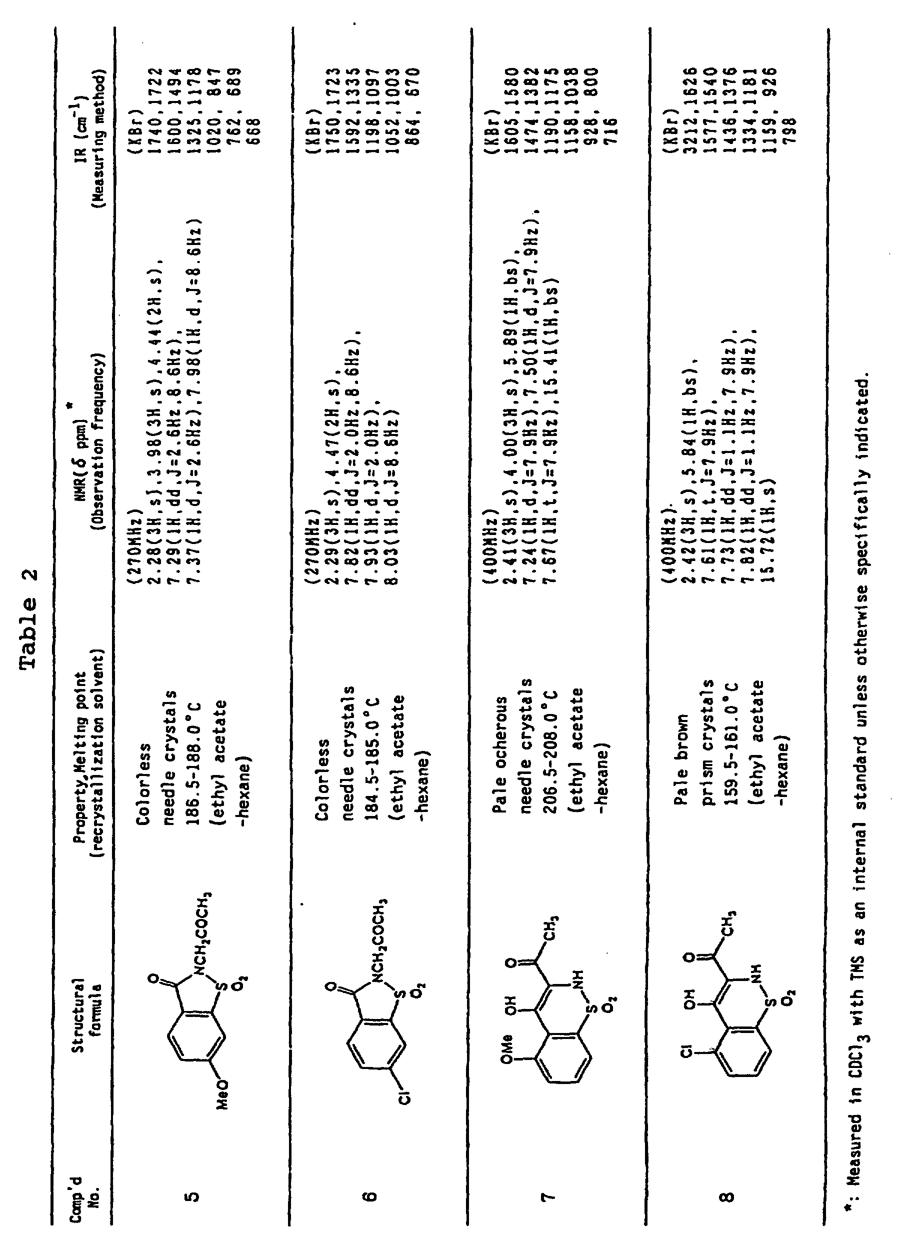

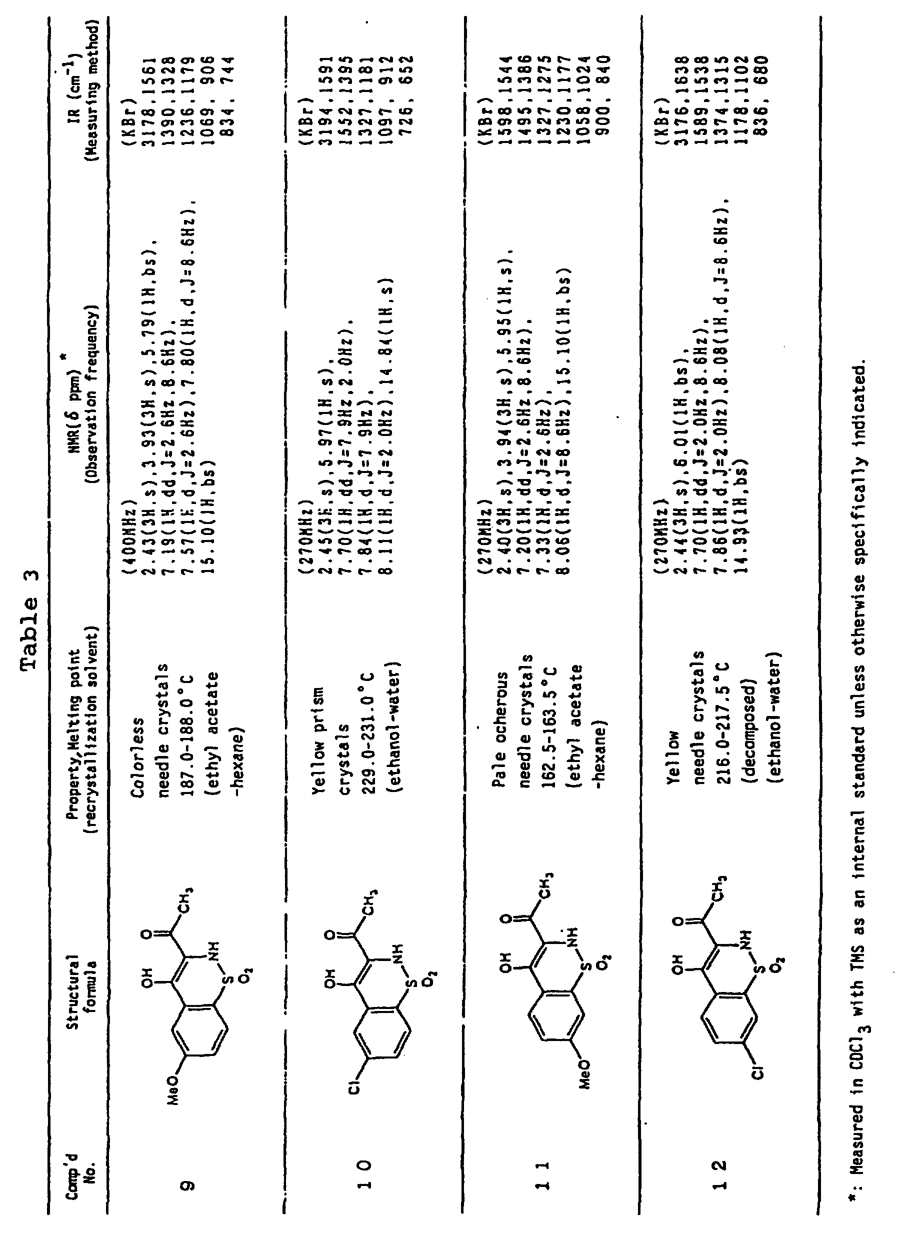

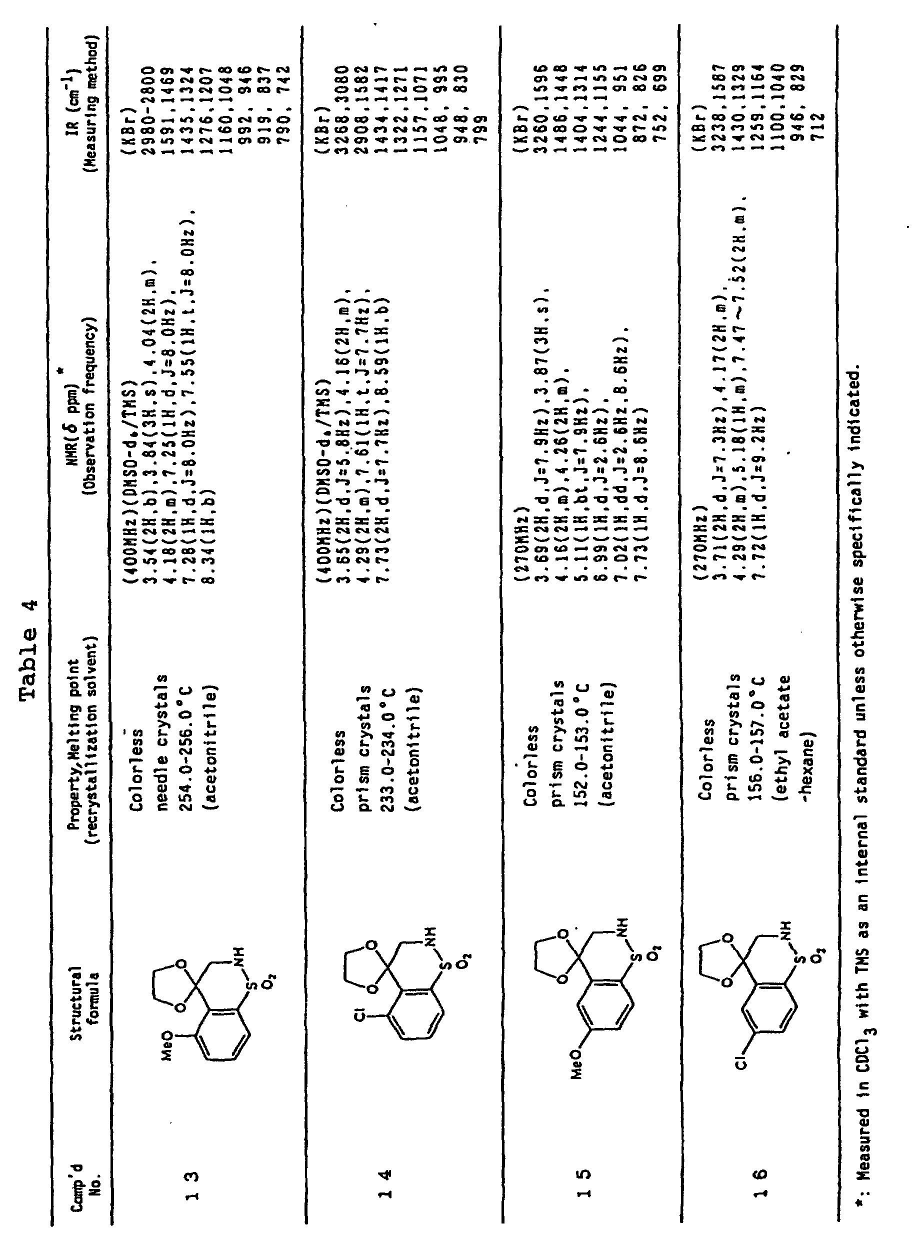

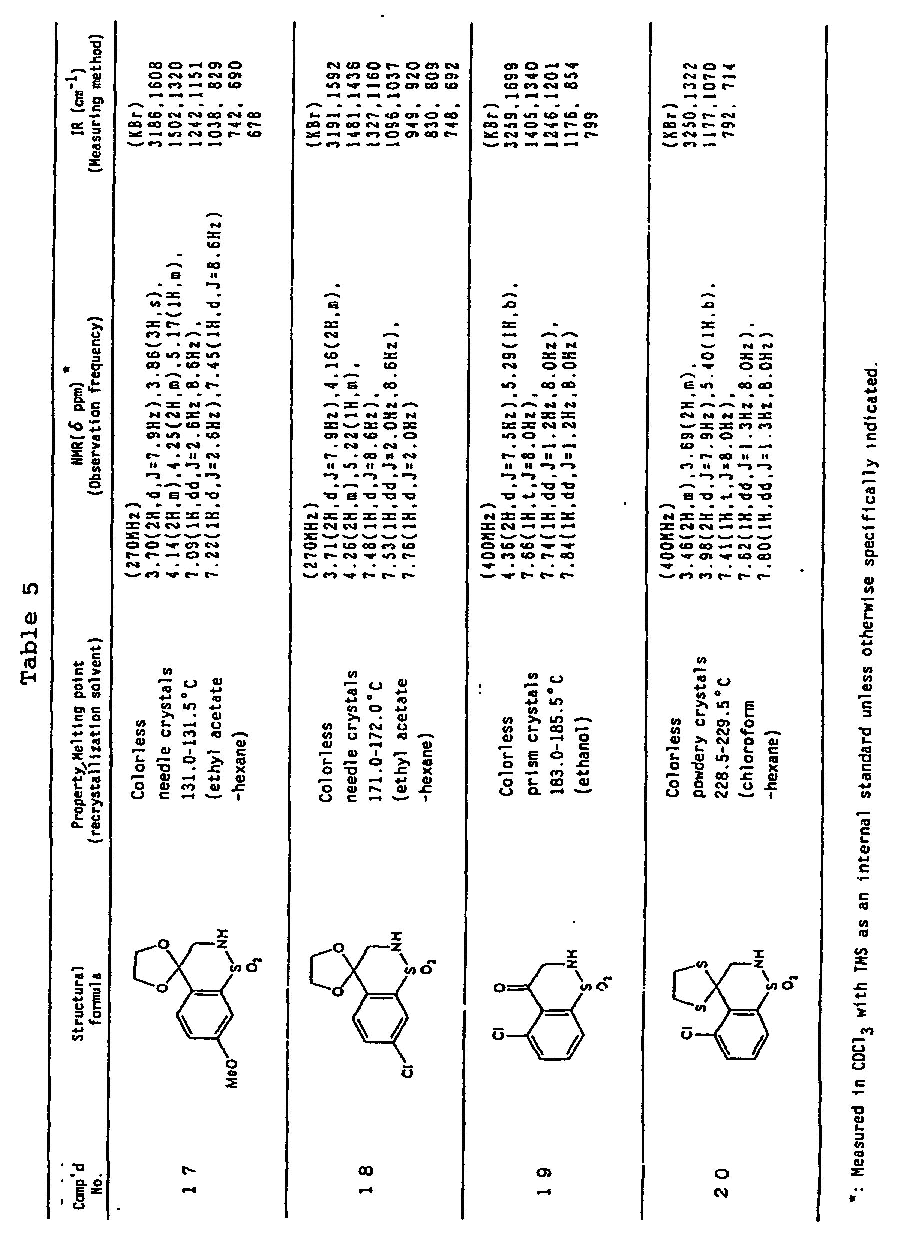

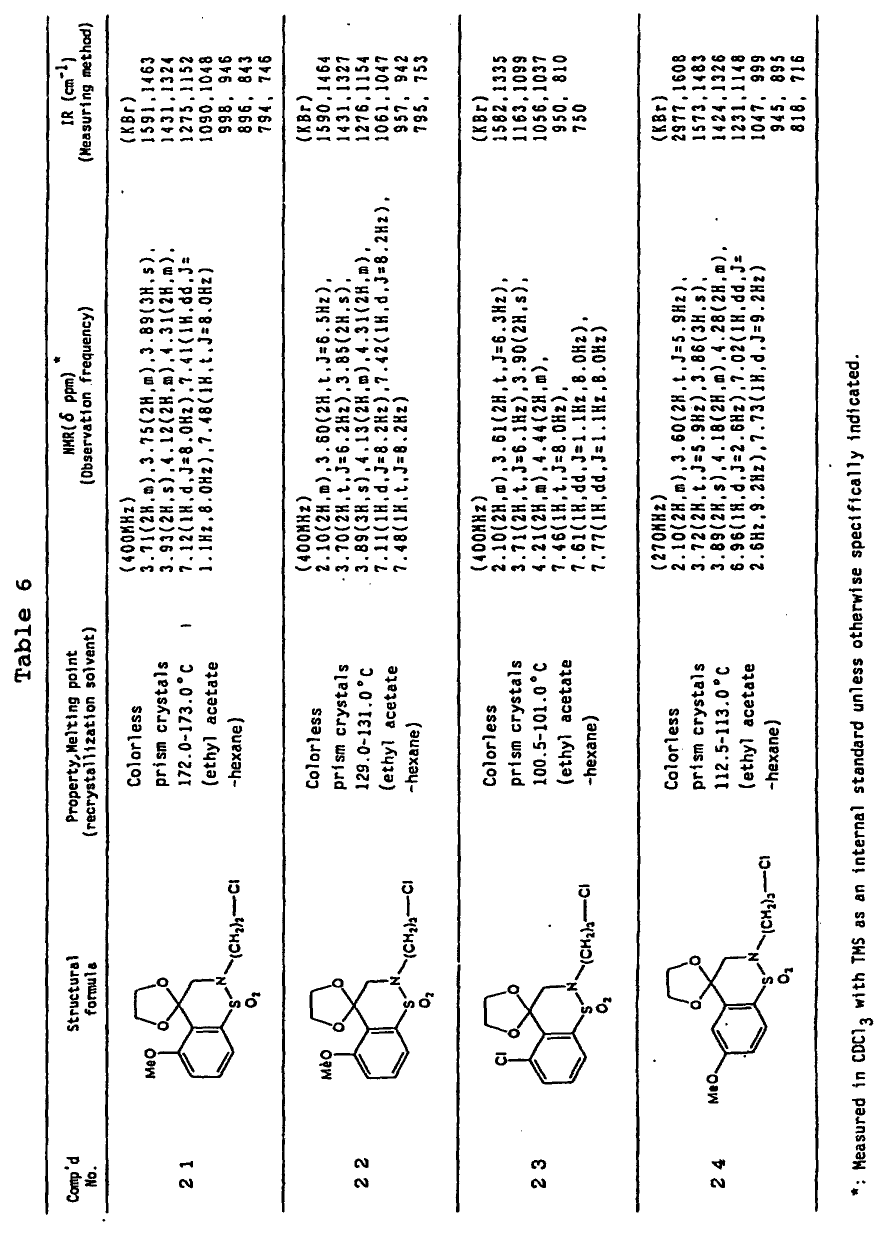

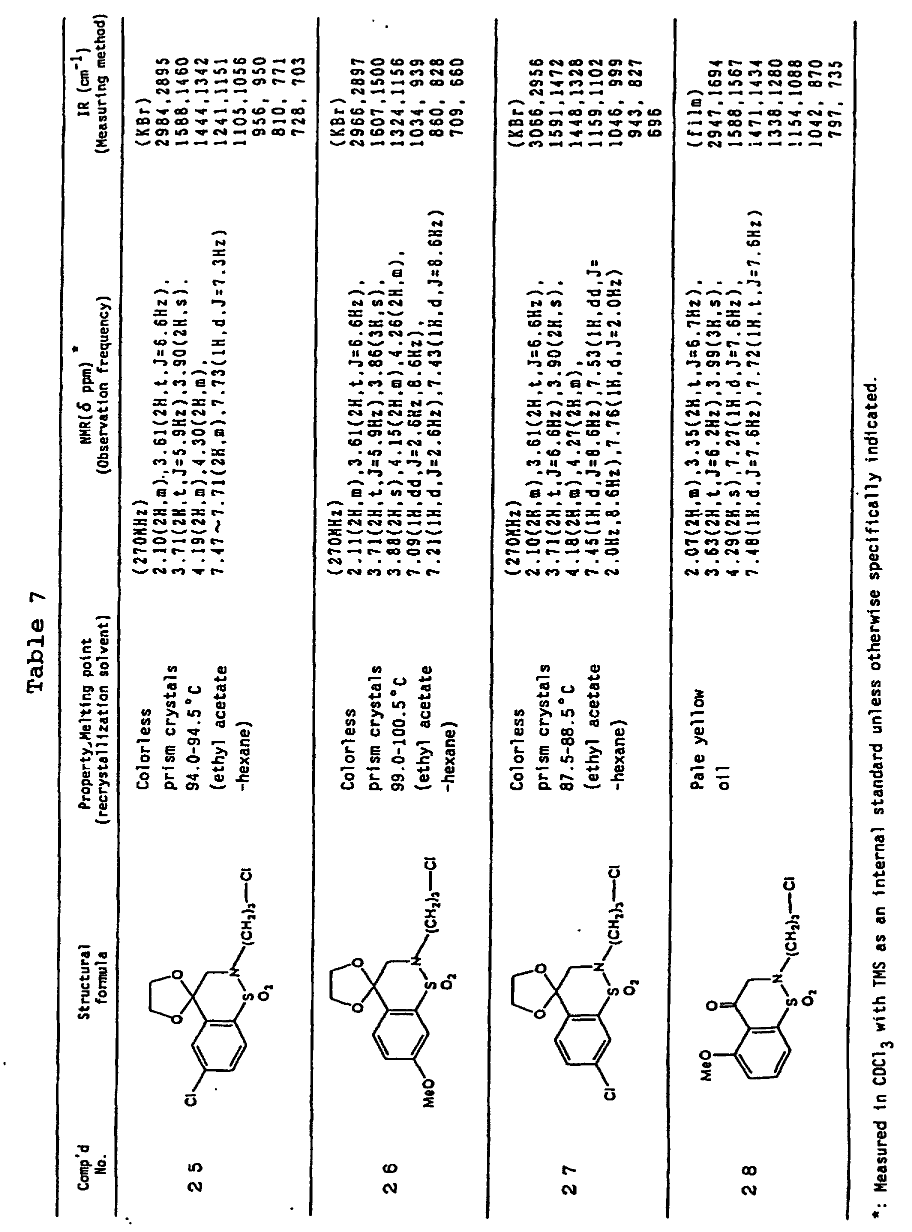

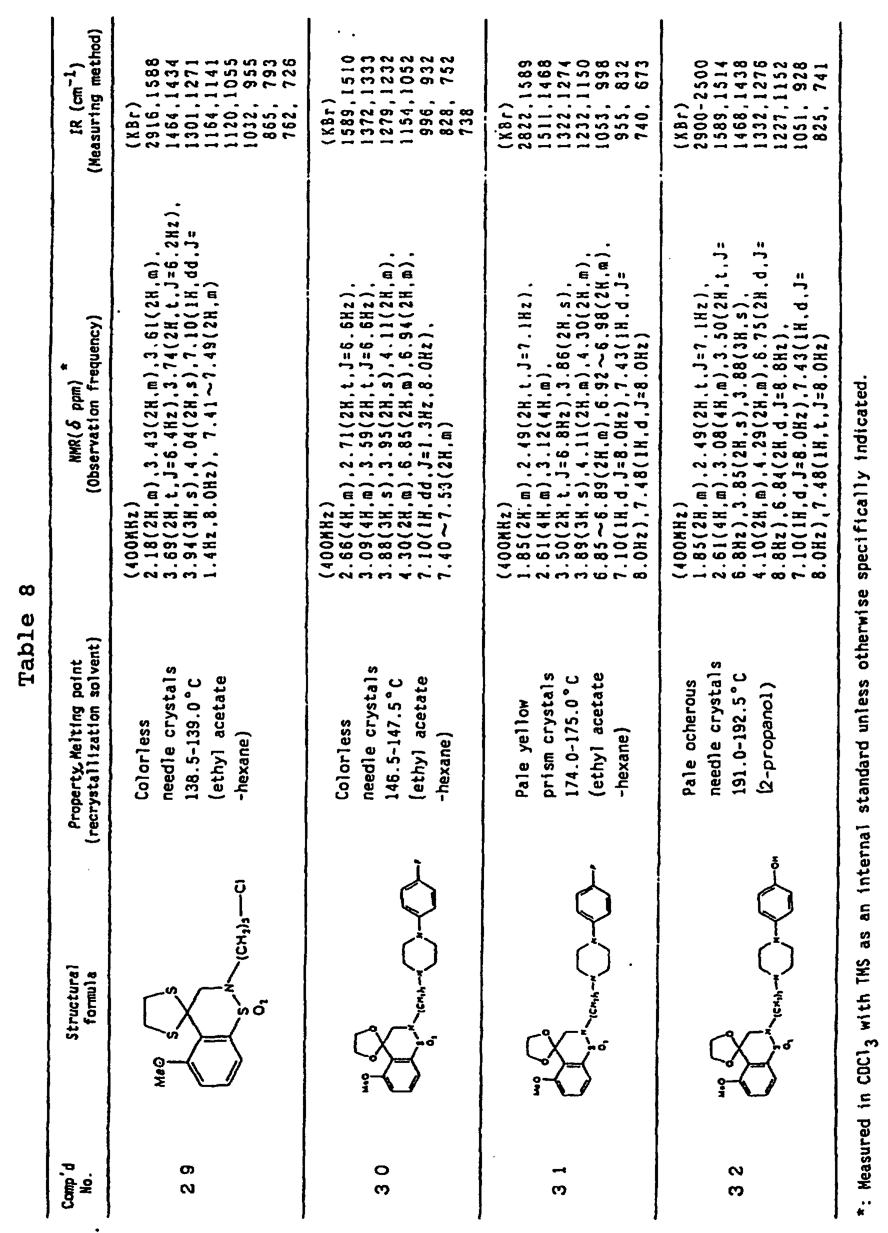

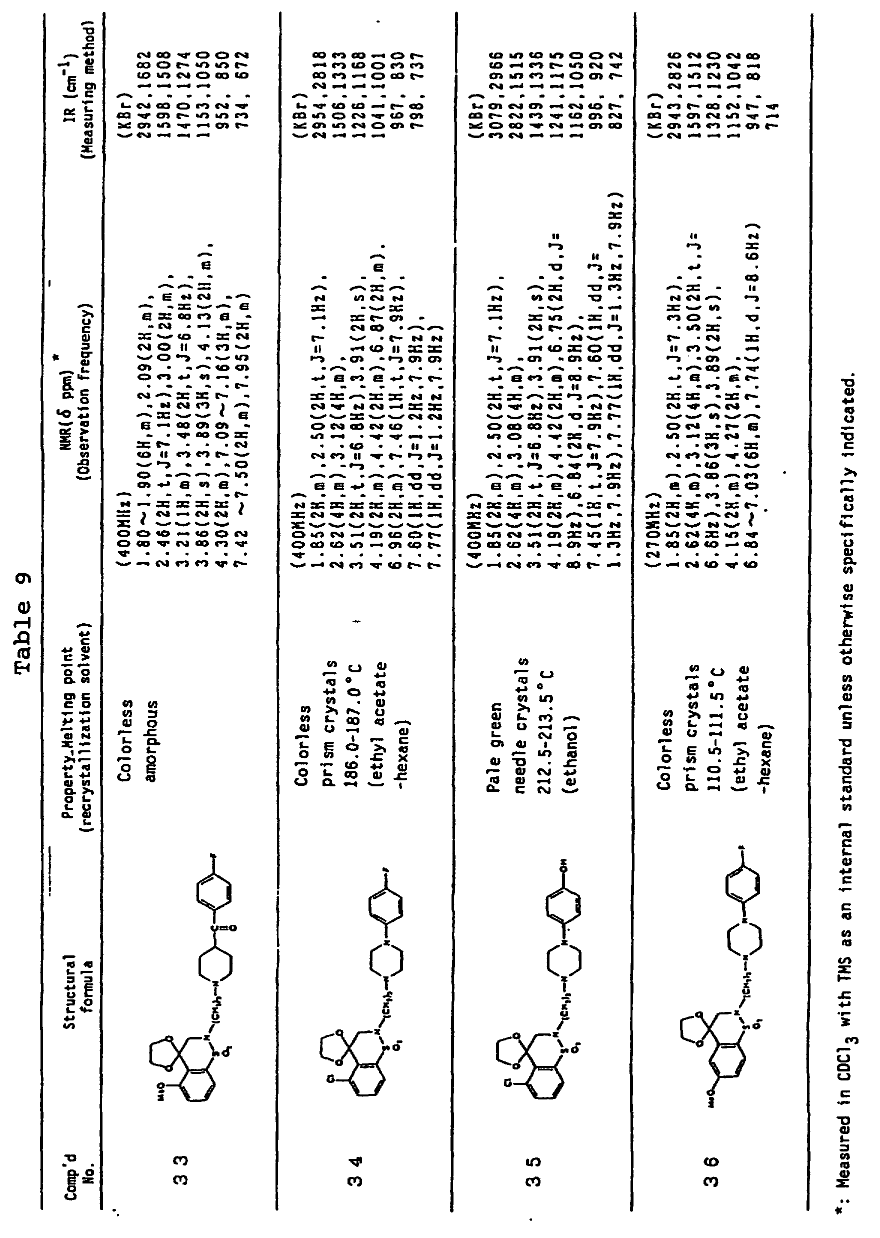

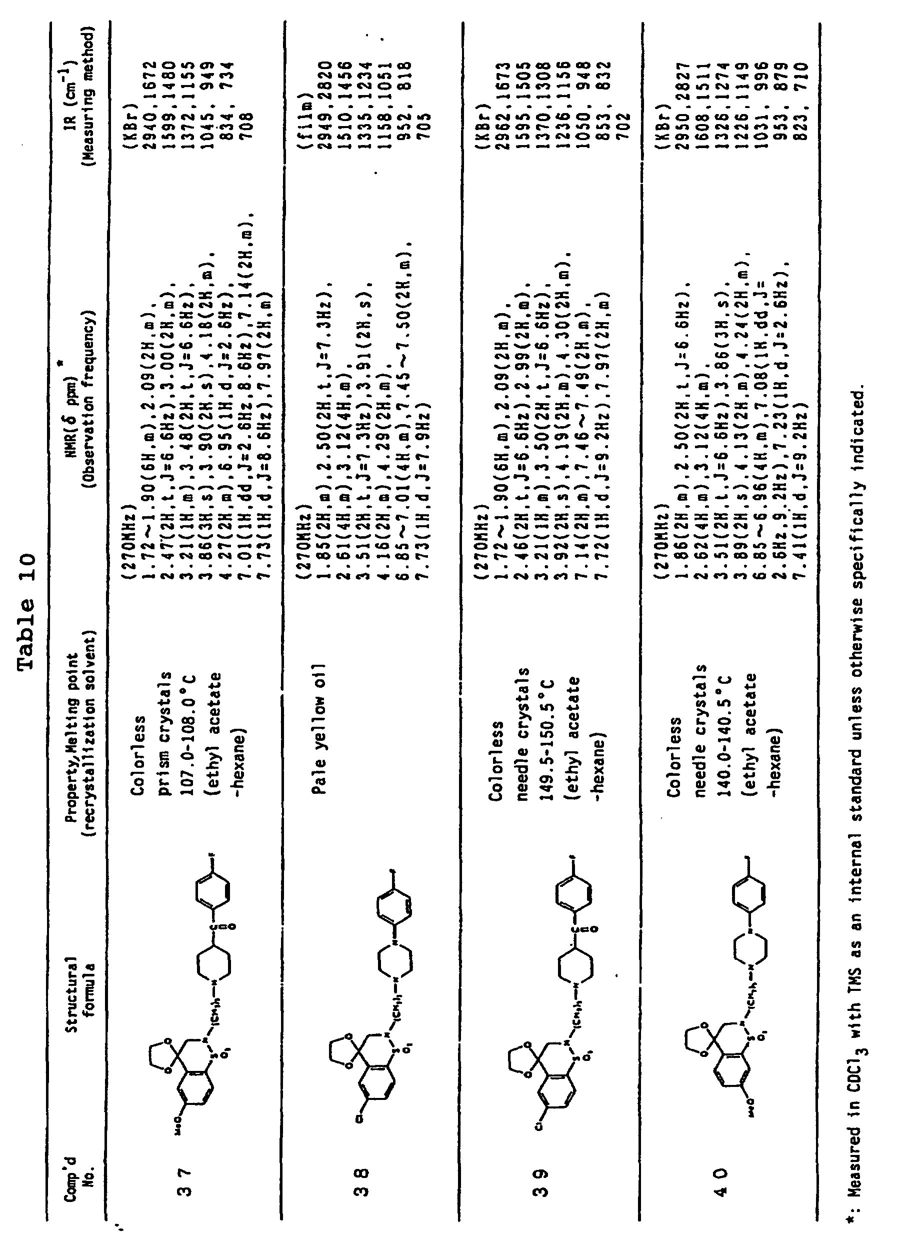

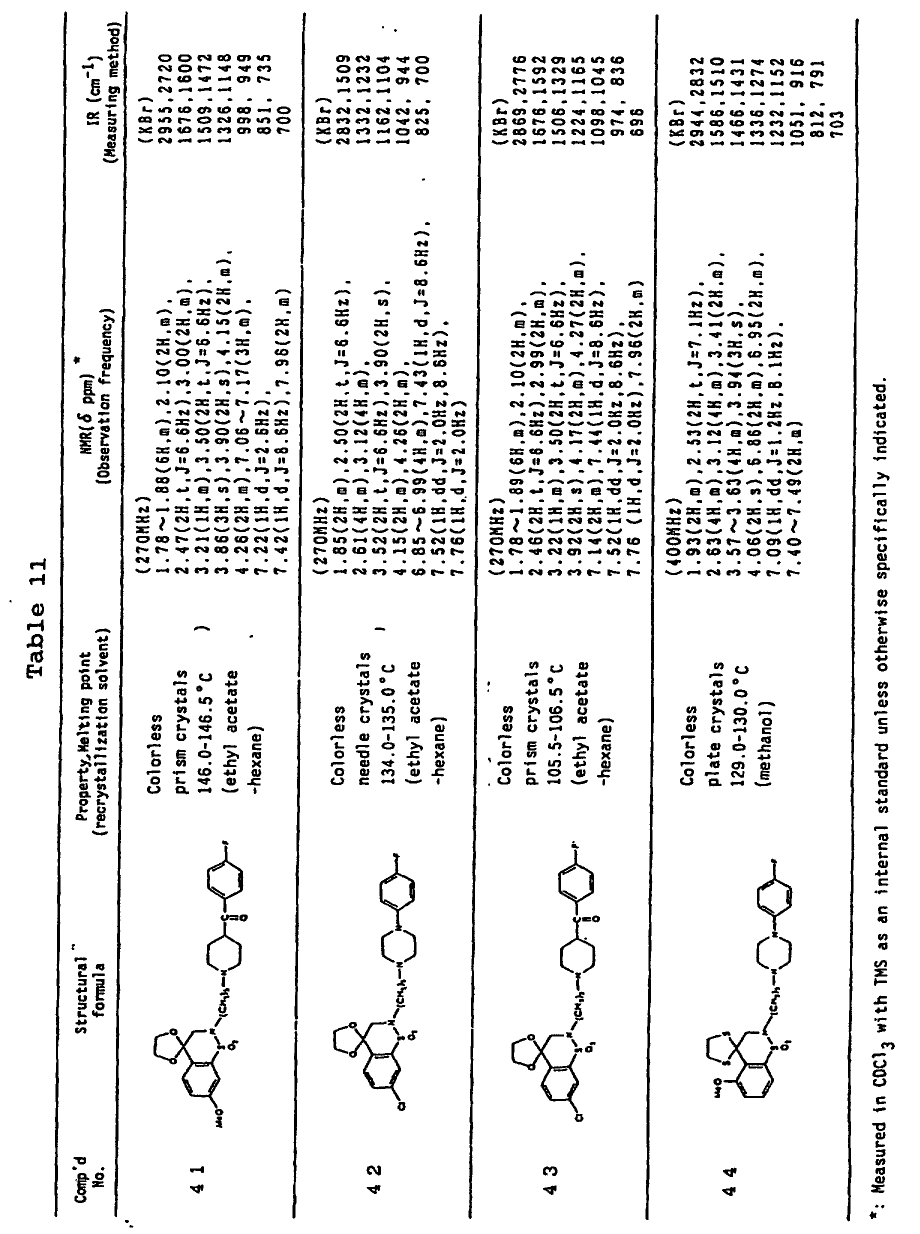

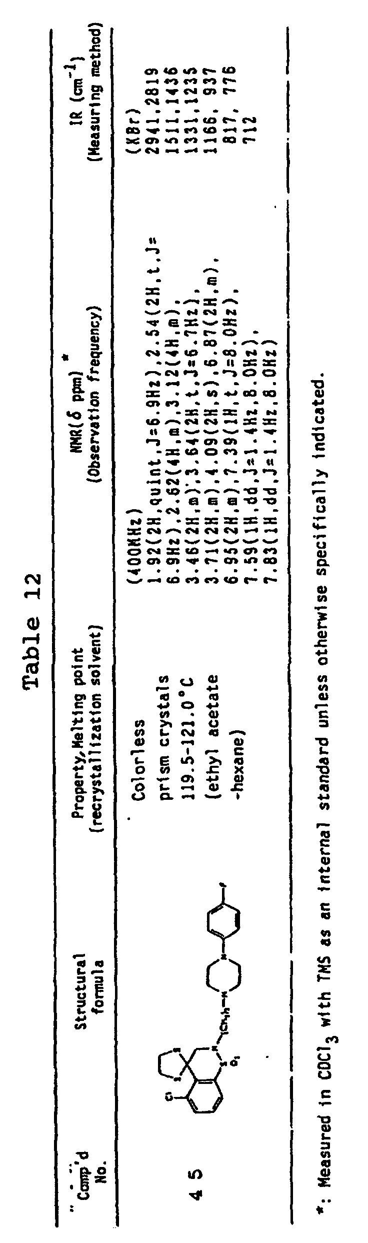

### Example 46

Following the process described in Example 31 or Example 32 or a process similar to it, the following compounds can each be obtained using as a substituted benzothiazine derivative, besides 2-(3-chloropropyl)-5-methoxy-3,4-dihydro-2H-1,2-benzothiazin-4-one 1,1-dioxide ethylene acetal (Compound No. 22), 2-(2-chloroethyl)-5-methoxy-3,4-dihydro-2H-1,2-benzothiazin-4-one 1,1-dioxide ethylene acetal, 2-(4-chlorobutyl)-5-methoxy-3,4-dihydro-2H-1,2-benzothiazin-4-one 1,1-dioxide ethylene acetal, 2-(3-chloropropyl)-5-methoxy-3,4-dihydro-2H-1,2-benzothiazine-4-spiro-2'-(1',3'-dithiolane) 1,1-dioxide, 2-(2-chloroethyl)-5-methoxy-3,4-dihydro-2H-1,2-benzothiazine-4-spiro-2'-(1',3'-dithiolane) 1,1-dioxide, 2-(4-chlorobutyl)-5-methoxy-3,4-dihydro-2H-1,2-benzothiazine-4-spiro-2'-(1',3'-dithiolane) 1,1-dioxide, 2-(3-chloropropyl)-4,4,5-trimethoxy-3,4-dihydro-2H-1,2-benzothiazine 1,1-dioxide, 2-(2-chloroethyl)-4,4,5-trimethoxy-3,4-dihydro-2H-1,2-benzothiazine 1,1-dioxide, 2-(4-chlorobutyl)-4,4,5-trimethoxy-3,4-dihydro-2H-1,2-benzothiazine 1,1-dioxide, 2-(3-chloropropyl)-5-methoxy-3,4-dihydro-2H-1,2-benzothiazine-4-spiro-2'-(1',3'-dioxane) 1,1-dioxide, 2-(3-chloropropyl)-5-methoxy-3,4-dihydro-2H-1,2-benzothiazine-4-spiro-2'-(1',3'-dithiane) 1,1-dioxide, 2-(3-chloropropyl)-4,4-bis(ethylthio)-5-methoxy-3,4-dihydro-2H-1,2-benzothiazine 1,1-dioxide, 2-(3-chloropropyl)-5-methoxy-3,4-dihydro-2H-1,2-benzothiazine-4-one 1,1-dioxide, 2-(3-chloropropyl)-4-hydroxy-5-methoxy-3,4-dihydro-2H-1,2-benzothiazine 1,1-dioxide, 2-(2-chloroethyl)-4-hydroxy-5-methoxy-3,4-dihydro-2H-1,2-benzothiazine 1,1-dioxide, or 2-(4-chlorobutyl)-4-hydroxy-5-methoxy-3,4-dihydro-2H-1,2-benzothiazine 1,1-dioxide, and as a piperazine derivative or piperidine derivative, 1-phenylpiperazine, 1-(2-fluorophenyl)piperazine, 1-(4-fluorophenyl)piperazine, 1-(4-hydroxyphenyl)piperazine, 1-(3-methoxyphenyl)piperazine, 1-(2-pyridyl)piperazine or 4-(4-fluorobenzoyl)piperidine.

Further, modifications to the substituted benzothiazine derivatives as raw materials and the treatments in the Examples make it possible to obtain corresponding compounds. For such modifications, the disclosure of PCT International Application No. PCT/JP94/02194 (now WO 95/18117) will be very useful.
(1) 2-[3-[4-(2-fluorophenyl)piperazin-1-yl]propyl]-5-methoxy-3,4-dihydro-2H-1,2-benzothiazin-4-one 1,1-dioxide ethylene acetal
(2) 2-[4-[4-(4-fluorophenyl)piperazin-1-yl]butyl]-5-methoxy-3,4-dihydro-2H-1,2-benzothiazin-4-one 1,1-dioxide ethylene acetal
(3) 5-methoxy-2-[3-(4-phenylpiperazin-1-yl)propyl]-3,4-dihydro-2H-1,2-benzothiazin-4-one 1,1-dioxide ethylene acetal
(4) 5-methoxy-2-[3-[4-(3-methoxyphenyl)piperazin-1-yl]propyl]-3,4-dihydro-2H-1,2-benzothiazin-4-one 1,1-dioxide ethylene acetal
(5) 5-methoxy-2-[3-[4-(2-pyridyl)piperazin-1-yl]propyl]-3,4-dihydro-2H-1,2-benzothiazin-4-one 1,1-dioxide ethylene acetal
(6) 2-[3-[4-(4-hydroxyphenyl)piperazin-1-yl]propyl]-5-methoxy-3,4-dihydro-2H-1,2-benzothiazine-4-spiro-2'-(1',3'-dithiolane) 1,1-dioxide
(7) 2-[3-[4-(4-fluorobenzoyl)piperidino]propyl]-5-methoxy-3,4-dihydro-2H-1,2-benzothiazine-4-spiro-2'-(1',3'-dithiolane) 1,1-dioxide
(8) 2-[3-[4-(2-fluorophenyl)piperazin-1-yl]propyl]-5-methoxy-3,4-dihydro-2H-1,2-benzothiazine-4-spiro-2'-(1',3'-dithiolane) 1,1-dioxide
(9) 2-[2-[4-(4-fluorophenyl)piperazin-1-yl]ethyl]-5-methoxy-3,4-dihydro-2H-1,2-benzothiazine-4-spiro-2'-(1',3'-dithiolane) 1,1-dioxide
(10) 2-[4-[4-(4-fluorophenyl)piperazin-1-yl]butyl]-5-methoxy-3,4-dihydro-2H-1,2-benzothiazine-4-spiro-2'-(1',3'-dithiolane) 1,1-dioxide
(11) 5-methoxy-2-[3-[4-(2-pyridyl)piperazin-1-yl]propyl]-3,4-dihydro-2H-1,2-benzothiazine-4-spiro-2'-(1',3'-dithiolane) 1,1-dioxide
(12) 2-[3-[4-(4-fluorophenyl)piperazin-1-yl]propyl]-4,4,5-trimethoxy-3,4-dihydro-2H-1,2-benzothiazine 1,1-dioxide
(13) 2-[3-[4-(4-fluorobenzoyl)piperidino]propyl]-4,4,5-trimethoxy-3,4-dihydro-2H-1,2-benzothiazine 1,1-dioxide
(14) 2-[3-[4-(2-fluorophenyl)piperazin-1-yl]propyl]-4,4,5-trimethoxy-3,4-dihydro-2H-1,2-benzothiazine 1,1-dioxide
(15) 2-[2-[4-(4-fluorophenyl)piperazin-1-yl]ethyl]-4,4,5-trimethoxy-3,4-dihydro-2H-1,2-benzothiazine 1,1-dioxide
(16) 2-[4-(4-(4-fluorophenyl)piperazin-1-yl]butyl]-4,4,5-trimethoxy-3,4-dihydro-2H-1,2-benzothiazine 1,1-dioxide
(17) 4,4,5-trimethoxy-2-[3-[4-(2-pyridyl)piperazin-1-yl]propyl]-3,4-dihydro-2H-1,2-benzothiazine 1,1-dioxide
(18) 2-[3-[4-(4-fluorophenyl)piperazin-1-yl]propyl]-5-methoxy-3,4-dihydro-2H-1,2-benzothiazine-4-spiro-2'-(1',3'-dioxane) 1,1-dioxide
(19) 2-[3-[4-(4-fluorobenzoyl)piperidino]propyl]-5-methoxy-3,4-dihydro-2H-1,2-benzothiazine-4-spiro-2'-(1',3'-dioxane) 1,1-dioxide
(20) 2-[3-[4-(2-fluorophenyl)piperazin-1-yl]propyl]-5-methoxy-3,4-dihydro-2H-1,2-benzothiazine-4-spiro-2'-(1',3'-dioxane) 1,1-dioxide
(21) 2-[2-[4-(4-fluorophenyl)piperazin-1-yl]ethyl]-5-methoxy-3,4-dihydro-2H-1,2-benzothiazine-4-spiro-2'-(1',3'-dioxane) 1,1-dioxide
(22) 2-[4-[4-(4-fluorophenyl)piperazin-1-yl]butyl]-5-methoxy-3,4-dihydro-2H-1,2-benzothiazine-4-spiro-2'-(1',3'-dioxane) 1,1-dioxide
(23) 5-methoxy-2-[3-[4-(2-pyridyl)piperazin-1-yl]propyl]-3,4-dihydro-2H-1,2-benzothiazine-4-spiro-2'-(1',3'-dioxane) 1,1-dioxide
(24) 2-[3-[4-(4-fluorophenyl)piperazin-1-yl]propyl]-5-methoxy-3,4-dihydro-2H-1,2-benzothiazine-4-spiro-2'-(1',3'-dithiane) 1,1-dioxide
(25) 2-[3-[4-(4-hydroxyphenyl)piperazin-1-yl]propyl]-5-methoxy-3,4-dihydro-2H-1,2-benzothiazine-4-spiro-2'-(1',3'-dithiane) 1,1-dioxide
(26) 2-[3-[4-(4-fluorobenzoyl)piperidino]propyl]-5-methoxy-3,4-dihydro-2H-1,2-benzothiazine-4-spiro-2'-(1',3'-dithiane) 1,1-dioxide
(27) 4,4-bis(ethylthio)-2-[3-[4-(4-fluorophenyl)piperazin-1-yl]propyl]-5-methoxy-3,4-dihydro-2H-1,2-benzothiazine 1,1-dioxide
(28) 4,4-bis(ethylthio)-2-[3-[4-(4-hydroxyphenyl)piperazin-1-yl]propyl]-5-methoxy-3,4-dihydro-2H-1,2-benzothiazine 1,1-dioxide
(29) 4,4-bis(ethylthio)-2-[3-[4-(4-fluorobenzoyl)piperidino]propyl]-5-methoxy-3,4-dihydro-2H-1,2-benzothiazine 1,1-dioxide
(30) 2-[3-[4-(4-fluorophenyl)piperazin-1-yl]propyl]-4-hydroxy-5-methoxy-3,4-dihydro-2H-1,2-benzothiazine 1,1-dioxide
(31) 4-hydroxy-2-[3-[4-(4-hydroxyphenyl)piperazin-1-yl]propyl]-5-methoxy-3,4-dihydro-2H-1,2-benzothiazine 1,1-dioxide
(32) 2-[3-[4-(4-fluorobenzoyl)piperidino]propyl]-4-hydroxy-5-methoxy-3,4-dihydro-2H-1,2-benzothiazine 1,1-dioxide
(33) 2-[3-[4-(2-fluorophenyl)piperazin-1-yl]propyl]-4-hydroxy-5-methoxy-3,4-dihydro-2H-1,2-benzothiazine 1,1-dioxide
(34) 2-[2-[4-(4-fluorophenyl)piperazin-1-yl]ethyl]-4-hydroxy-5-methoxy-3,4-dihydro-2H-1,2-benzothiazine 1,1-dioxide
(35) 2-[4-[4-(4-fluorophenyl)piperazin-1-yl]butyl]-4-hydroxy-5-methoxy-3,4-dihydro-2H-1,2-benzothiazine 1,1-dioxide
(36) 4-hydroxy-5-methoxy-2-[3-[4-(2-pyridyl)piperazin-1-yl]propyl]-3,4-dihydro-2H-1,2-benzothiazine 1,1-dioxide

### Test

With respect to the compounds of the present invention, their anti-serotonin (5-HT) action and anti-α₁ action were investigated by the testing methods which will be described below. The results of some representative compounds are shown in Table 13.

### (1) Anti-serotonin action (anti-5-HT action)

The superior mesenteric artery of each Hartley male guinea pig (body weight: 300-500 g) was excised. A preparation cut in a helical form was suspended under 0.3 g load in a Magnus cylinder filled with the Tyrode solution which had been aerated with a gas mixture of 95% O₂ + 5% CO₂ and maintained at 37°C. Using an isometric transducer ("UL-10", manufactured by SHINKOH K.K.) and a pressure preamplifier ("DSA-605A", manufactured by SHINKOH K.K.), variations in tension were measured. The isometric tensions were recorded on a pen-writing recorder ("VP-6537A", manufactured by NATIONAL K.K.). Taking the contraction induced by 10⁻⁵ M serotonin (5-HT) as 100%, the percent contractions in the presence of each test drug at 10⁻⁷ and 10⁻⁶ M were determined as anti-5-HT action.

### (2) Anti-α₁ action

The thoracic aorta of each Hartley male guinea pig (body weight: 300-500 g) was excised. A preparation cut in a helical form was suspended under 1 g load in a Magnus cylinder filled with the Tyrode solution which had been aerated with a gas mixture of 95% O₂ + 5% CO₂ and maintained at 37°C. Using an isometric transducer ("TB-612J", manufactured by NIHON KOHDEN) and a pressure preamplifier ("AP-620G", manufactured by NIHON KOHDEN), variations in tension were measured. The isometric tensions were recorded on a thermal pen-writing recorder ("WT-647G", manufactured by NIHON KOHDEN).

Taking the tonic contraction induced by 10⁻⁵ M norepinephrine (NE) as 100%, the percent contractions upon addition of each test drug at 10⁻⁸ and 10⁻⁷ M were determined as anti-α₁ action.

### (Results)

**Table 13**

| Comp'd No. | Anti 5-HT action (% of Control) | | Anti α₁ action (% of Control) | |
|---|---|---|---|---|
| | 10⁻⁷M | 10⁻⁶M | 10⁻⁸M | 10⁻⁷M |
| 30 | 10.5 | NT | 99.3 | 96.1 |
| 31 | 5.6 | NT | 100 | 81.1 |
| 32 | 10.9 | NT | 100.9 | 98.6 |
| 33 | 17.1 | NT | 99.7 | 90.0 |
| 34 | 49.5 | NT | 98.3 | 89.3 |
| 35 | 23.5 | NT | 101.1 | 98.9 |
| 36 | 63.0 | 14.1 | 100.4 | 62.3 |
| 37 | 40.2 | 13.9 | 100.7 | 88.3 |
| 38 | 48.0 | 23.6 | 97.1 | 80.2 |
| 40 | 39.0 | 9.2 | 96.6 | 73.6 |
| 41 | 51.6 | 20.3 | 93.2 | 71.6 |
| 44 | 49.3 | NT | 97.9 | 13.0 |
| NT: Not tested. | | | | |

## Claims

1. A substituted benzothiazine derivative represented by the following formula (I): 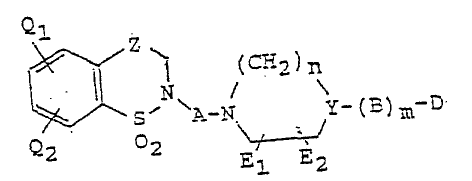 wherein Z represents one of the following groups: 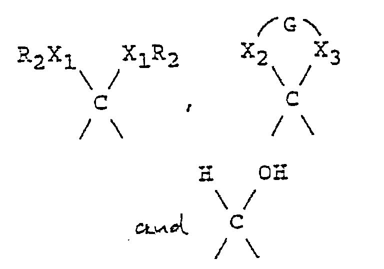 wherein R₂ represents a C₁-C₄ alkyl group, a C₆-C₁₄ aryl group, or a C₇-C₂₂ aralkyl group, these groups being optionally substituted by one or more substituents chosen from halogen atoms, C₁-C₄ alkyl groups and/or C₁-C₄ alkoxy groups; X₁, X₂ and X₃ each independently represents an oxygen atom or a sulfur atom, G represents an ethylene group with one or more of the hydrogen atoms thereof optionally substituted by a like number of halogen atoms and/or C₁-C₄ alkyl, C₆-C₁₄ aryl, C₇-C₂₂ aralkyl and/or C₁-C₄ alkylidene groups or a trimethylene group with one or more of the hydrogen atoms thereof optionally substituted by a like number of halogen atoms and/or C₁-C₄ alkyl, C₆-C₁₄ aryl, C₇-C₂₂ aralkyl and/or C₁-C₄ alkylidene groups, provided that G is not an ethylene group if both X₂ and X₃ are oxygen atoms,
Q₁ represents a hydrogen atom, a hydroxyl group, a halogen atom, a C₁-C₄ alkyl group, a C₁-C₄ alkoxy group,
Q₂ represents a hydroxyl group, a halogen atom, a C₁-C₄ alkyl group, a C₁-C₄ alkoxy group,
A represents a C₂-C₁₀ alkylene group,
Y represents CH or a nitrogen atom, m stands for 0 or 1, n stands for 2, and B represents a carbonyl group,
E₁ and E₂ each independently represents a hydrogen atom or a C₁₋₄ alkyl group, and D represents a phenyl, pyridyl, pyrimidinyl, benzoisothiazolyl, benzisoxazolyl or indolyl group, wherein one or more of the hydrogen atoms are optionally substituted with a halogen, C₁₋₄ alkyl, C₁₋₄ alkoxy, cyano, nitro, carboxyl, C₁₋₆ alkoxycarbonyl, C₁₋₄ alkylsulfonylamino, carbamoyl or hydroxyl group.

2. A substituted benzothiazine derivative or a salt thereof according to claim 1, wherein in the formula (I), Z represents the following group: 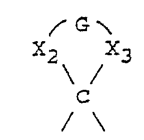 wherein G, X₂ and X₃ have the same meanings as defined in claim 1.

3. A substituted benzothiazine derivative or a salt thereof according to claim 1, wherein in the formula (I), Z represents the following group:

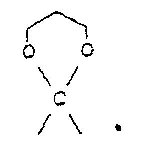

4. A substituted benzothiazine derivative or a salt thereof according to claim 1, wherein in the formula (I), Z represents the following group:

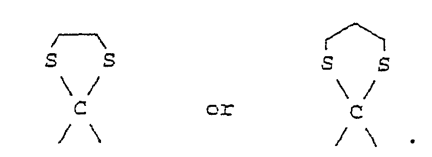

5. A substituted benzothiazine derivative or a salt thereof according to claim 1, wherein in the formula (I), Z represents the following group:

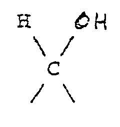

6. A substituted benzothiazine derivative or a salt thereof according to any one of claims 1, 2, 3, 4 and 5, wherein in the formula (I), Q₁ represents a hydrogen atom and Q₂ represents a methoxy group.

7. A substituted benzothiazine derivative or a salt thereof according to any one of claims 1, 2, 3, 4, 5, and 6, wherein in the formula (I), A represents an ethylene group or a trimethylene group.

8. A substituted benzothiazine derivative or a salt thereof according to any one of claims 1, 2, 3, 4, 5, 6, and 7 wherein in the formula (I), Y represents CH and D represents a substituted or unsubstituted phenyl group.

9. A substituted benzothiazine derivative or a salt thereof according to any one of claims 1, 2, 3, 4, 5, 6, or 7, wherein in the formula (I), Y represents a nitrogen atom, m stands for 0, and D represents a substituted or unsubstituted phenyl group.

10. A substituted benzothiazine derivative or a salt thereof according to any one of claims 1, 2, 3, 4, 5, 6, 7, 8, or 9, wherein in the formula (I), E₁ and E₂ each represents a hydrogen atom.

11. A process for the preparation of a substituted benzothiazine derivative of claim 1 represented by the following formula (Ia) : 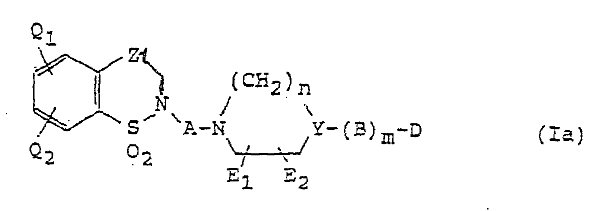 wherein
Z₁ represents one of the following groups: 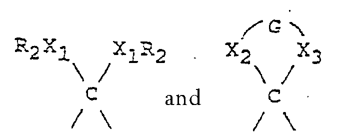 in which
G, R₂, X₁, X₂, X₃ A, B, D, E₁, E₂, Q₁, Q₂, Y, m and n have the same meanings as defined in claim 1, which comprises:
reacting a compound, which is represented by the following formula (II) : 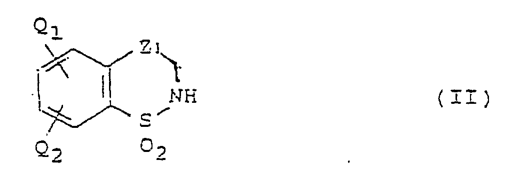 wherein Q₁, Q₂ and Z₁ have the same meanings as defined above, with a compound represented by the following formula (III) :
W-A-W' (III)
wherein A has the same meaning as defined above and W and W' may be the same or different and individually represent a substituent easily replaceable with an amino group, to obtain a compound represented by the following formula (IV) : 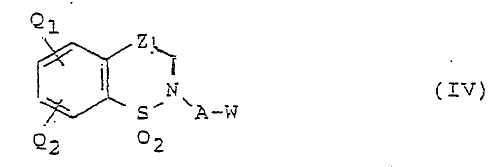 wherein A, Q₁, Q₂, W and Z₁ have the same meanings as defined above; and
then reacting the resulting compound with a nitrogen-containing compound represented by the following formula (V) : 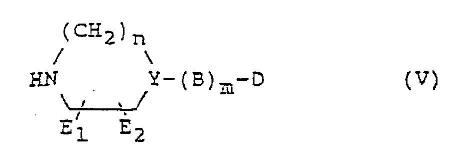 wherein B, D, E₁, E₂, Y, m and n have the same meanings as defined above.

12. A process for the preparation of a substituted benzothiazine derivative of claim 1 represented by the following formula (Ia) : 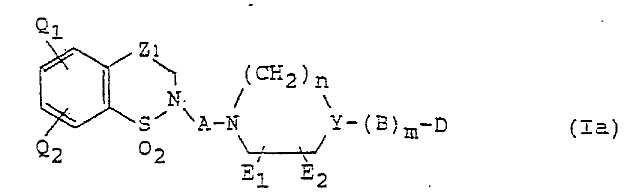 wherein A, B, D, E₁, E₂, Q₁, Q₂, Y, m and n have the same meanings as defined in claim 1 and Z₁ as defined in claim 11, which comprises:
reacting a compound, which is represented by the following formula (II) : 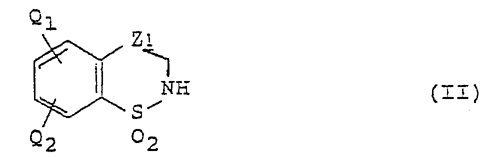 Wherein Q₁, Q₂ and Z₁ have the same meanings as defined above, with a nitrogen-containing compound represented by the following formula (VI) : 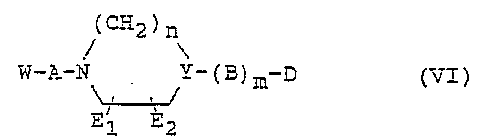
wherein A, B, D, E₁, E₂, Y, m and n have the same meanings as defined in claim 1 and W as defined in claim 11.

13. A process for the preparation of a substituted benzothiazine derivative of claim 1 represented by the following formula (If) : 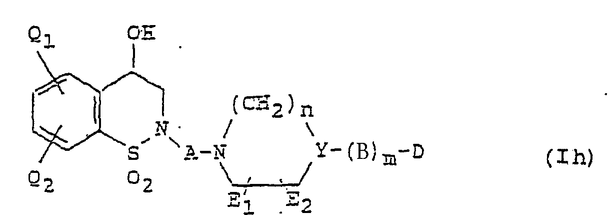 wherein A, B, D, E₁, E₂, Q₁, Q₂, Y, m and n have the same meanings as defined in claim 1, which comprises subjecting a substituted benzothiazine derivative represented by the following formula (Ic) : 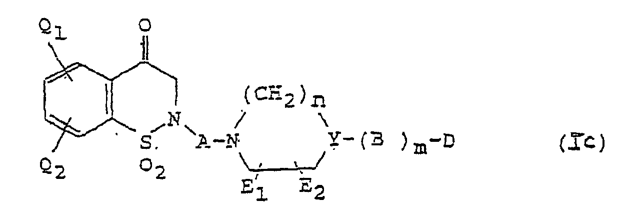 wherein A, B, D, E₁, E₂, Q₁, Q₂, Y, m and n have the same meanings as defined above, to reduction.

14. A process for the preparation of a substituted benzothiazine derivative of claim 1 represented by the following formula (Ih) : 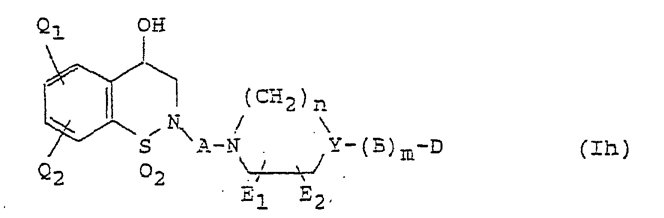 wherein A, B, D, E₁, E₂, Q₁, Q₂, Y, m and n have the same meanings as defined in claim 1, which comprises subjecting a compound, which is represented by the following formula (VIII) : 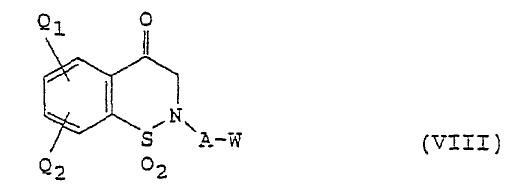 wherein A, Q₁, and Q₂ have the same meanings as defined in claim 1 and W as in claim 11, to reduction to obtain a compound represented by the following formula (X) : 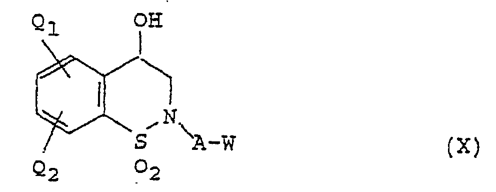 wherein A, Q₁, Q₂ and W have the same meanings as defined above, and then
reacting the resulting compound with a nitrogen-containing compound represented by the following formula (V) : 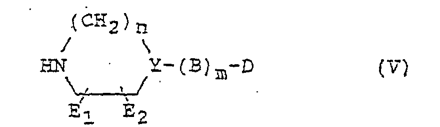 wherein B, D, E₁, E₂, Y, m and n have the same meanings as defined in claim 1.

15. An intermediate suitable for use in the production of a pharmaceutical, said intermediate being represented by the following formula (XX) : 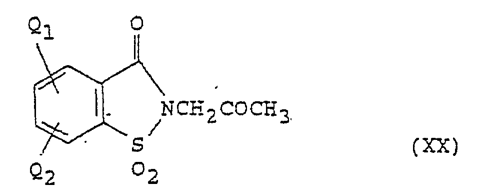 wherein Q₁ and Q₂ have the same meanings as defined in claim 1.

16. An intermediate suitable for use in the production of a pharmaceutical, said intermediate being represented by the following formula (XXI) : 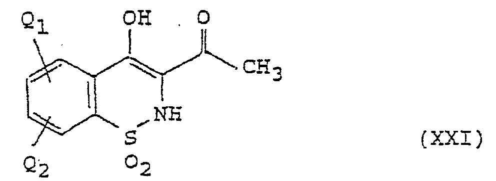 wherein Q₁ and Q₂ have the same meanings as defined in claim 1.

17. An intermediate suitable for use in the production of a pharmaceutical, said intermediate being represented by the following formula (II) : 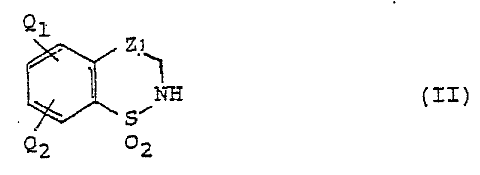 wherein Q₁ and Q₂ have the same meanings as defined in claim 1 and Z₁ is 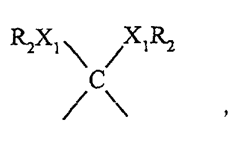 with X₁ and R₂ being defined as in claim 1.

18. An intermediate suitable for use in the production of a pharmaceutical, said intermediate being represented by the following formula (XXIZ) : 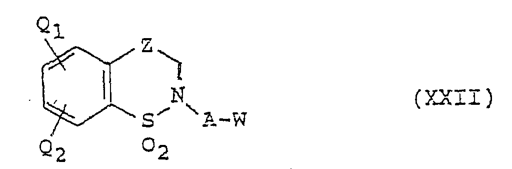 wherein A, Q₁, and Q₂, have the same meanings as defined in claim 1, W as in claim 11 and Z is 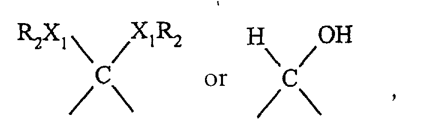 with X₁ and R₂ being defined as in claim 1.

19. A pharmaceutical comprising as an effective ingredient a substituted benzothiazine derivative or a salt thereof according to any of claims 1 to 10, or respective compounds wherein G is an ethylene group if both X₂ and X₃ are oxygen atoms.

20. Use of a substituted benzothiazine derivative according to any of claims 1 to 10 or respective compounds wherein G is an ethylene group if both X₂ and X₃ are oxygen atoms for the preparation of a pharmaceutical composition acting as serotonin-2-receptor antagonist.

21. Use of a substituted benzothiazine derivative according to any of claims 1 to 10 or respective compounds wherein G is an ethylene group if both X₂ and X₃ are oxygen atoms for the preparation of a pharmaceutical composition acting as a therapeutic for circulatory diseases.

## Patentansprüche

1. Substituiertes Benzothiazinderivat, dargestellt durch die nachstehende Formel (I): 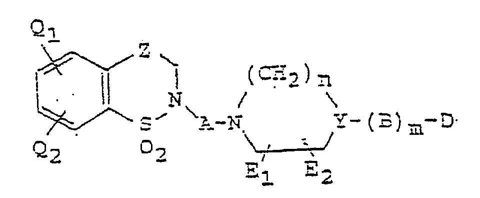 wobei Z einen der nachstehenden Reste bedeutet: 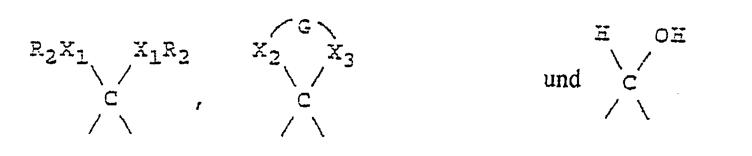 wobei R₂ einen C₁-C₄-Alkylrest, einen C₆-C₁₄-Arylrest oder einen C₇-C₂₂-Aralkylrest bedeutet, diese Reste gegebenenfalls durch einen oder mehrere Substituenten, ausgewählt aus Halogenatomen, C₁-C₄-Alkylresten und/oder C₁-C₄-Alkoxyresten, substituiert sind; X₁, X₂ und X₃ jeweils unabhängig ein Sauerstoffatom oder ein Schwefelatom bedeuten, G einen Ethylenrest, wobei eines oder mehrere der Wasserstoffatome davon gegebenenfalls durch die gleiche Anzahl an Halogenatomen und/oder C₁-C₄-Alkyl-, C₆-C₁₄-Aryl-, C₇-C₂₂-Aralkyl- und/oder C₁-C₄-Alkylidenresten substituiert sind oder einen Trimethylenrest, wobei eines oder mehrere der Wasserstoffatome davon gegebenenfalls durch die gleiche Anzahl an Halogenatomen und/oder C₁-C₄-Alkyl-, C₆-C₁₄-Aryl-, C₇-C₂₂-Aralkyl- und/oder C₁-C₄-Alkylidenresten substituiert sind, bedeutet, mit der Maßgabe, dass G kein Ethylenrest ist, falls X₂ und X₃ beides Sauerstoffatome sind,
Q₁ ein Wasserstoffatom, eine Hydroxylgruppe, ein Halogenatom, einen C₁-C₄-Alkylrest oder einen C₁-C₄-Alkoxyrest bedeutet,
Q₂ eine Hydroxylgruppe, ein Halogenatom, einen C₁-C₄-Alkylrest oder einen C₁-C₄-Alkoxyrest bedeutet,
A einen C₂-C₁₀-Alkylenrest bedeutet,
Y CH oder ein Stickstoffatom bedeutet, m für 0 oder 1 steht, n für 2 steht und B eine Carbonylgruppe bedeutet,
E₁ und E₂ jeweils unabhängig ein Wasserstoffatom oder einen C₁₋₄-Alkylrest bedeutet und D einen Phenyl-, Pyridyl-, Pyrimidinyl-, Benzoisothiazolyl-, Benzoisoxazolyl- oder Indolylrest bedeutet, wobei eines oder mehrere der Wasserstoffatome gegebenenfalls durch ein Halogenatom, einen C₁₋₄-Alkylrest, einen C₁₋₄-Alkoxyrest, eine Cyanogruppe, eine Nitrogruppe, eine Carboxylgruppe, einen C₁₋₆-Alkoxycarbonylrest, einen C₁₋₄-Alkylsulfonylaminorest, eine Carbamoylgruppe oder eine Hydroxylgruppe substituiert sind.

2. Substituiertes Benzothiazinderivat oder ein Salz davon gemäß Anspruch 1, wobei in der Formel (I) Z den nachstehenden Rest bedeutet: 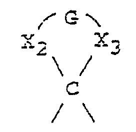 wobei G, X₂ und X₃ die gleiche Bedeutung haben, wie in Anspruch 1 definiert.

3. Substituiertes Benzothiazinderivat oder ein Salz davon gemäß Anspruch 1, wobei in der Formel (I) Z den nachstehenden Rest bedeutet:

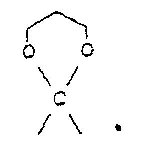

4. Substituiertes Benzothiazinderivat oder ein Salz davon gemäß Anspruch 1, wobei in der Formel (I) Z den nachstehenden Rest bedeutet:

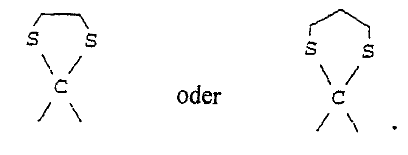

5. Substituiertes Benzothiazinderivat oder ein Salz davon gemäß Anspruch 1, wobei in der Formel (I) Z den nachstehenden Rest bedeutet:

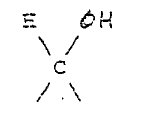

6. Substituiertes Benzothiazinderivat oder ein Salz davon gemäß einem der Ansprüche 1, 2, 3, 4 und 5, wobei in der Formel (I) Q₁ ein Wasserstoffatom bedeutet und Q₂ eine Methoxygruppe bedeutet.

7. Substituiertes Benzothiazinderivat oder ein Salz davon gemäß einem der Ansprüche 1, 2, 3, 4, 5 und 6, wobei in der Formel (I) A eine Ethylengruppe oder eine Trimethylengruppe bedeutet.

8. Substituiertes Benzothiazinderivat oder ein Salz davon gemäß einem der Ansprüche 1, 2, 3, 4, 5, 6 und 7, wobei in der Formel (I) Y CH bedeutet und D einen substituierten oder unsubstituierten Phenylrest bedeutet.

9. Substituiertes Benzothiazinderivat oder ein Salz davon gemäß einem der Ansprüche 1, 2, 3, 4, 5, 6 oder 7, wobei in der Formel (I) Y ein Stickstoffatom bedeutet, m für 0 steht und D einen substituierten oder unsubstituierten Phenylrest bedeutet.

10. Substituiertes Benzothiazinderivat oder ein Salz davon gemäß einem der Ansprüche 1, 2, 3, 4, 5, 6, 7, 8 oder 9, wobei in der Formel (I) E₁ und E₂ jeweils ein Wasserstoffatom bedeutet.

11. Verfahren zur Herstellung eines substituierten Benzothiazinderivats nach Anspruch 1, dargestellt durch die nachstehende Formel (Ia): 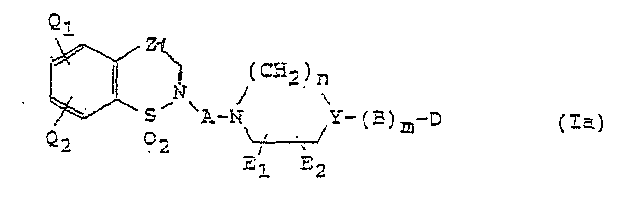 wobei Z₁ für einen der nachstehenden Reste steht: 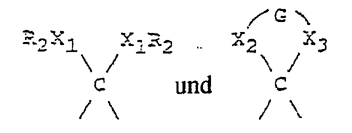 in welchen G, R₂, X₁, X₂, X₃, A, B, D, E₁, E₂, Q₁, Q₂, Y, m und n die gleiche Bedeutung haben, wie in Anspruch 1 definiert, welches umfasst:
Umsetzen einer Verbindung, welche durch die nachstehende Formel (II) dargestellt ist: 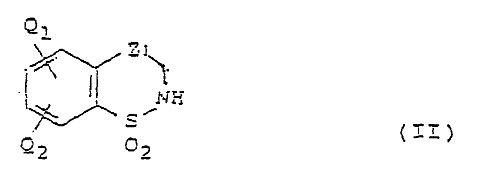 wobei Q₁, Q₂ und Z₁ die gleiche Bedeutung haben, wie vorstehend definiert, mit einer Verbindung, dargestellt durch die nachstehende Formel (III):
W―A―W' (III)
wobei A die gleiche Bedeutung hat, wie vorstehend definiert und W und W' gleich oder verschieden sein können und einzeln einen Substituenten bedeuten, welcher leicht durch eine Aminogruppe ersetzbar ist, um eine Verbindung, dargestellt durch die nachstehende Formel (IV) zu erhalten: 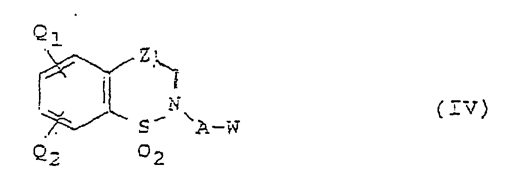 wobei A, Q₁, Q₂, W und Z₁ die gleiche Bedeutung haben, wie vorstehend definiert; und
dann Umsetzen der erhaltenen Verbindung mit einer Stickstoff enthaltenden Verbindung, dargestellt durch die nachstehende Formel (V): 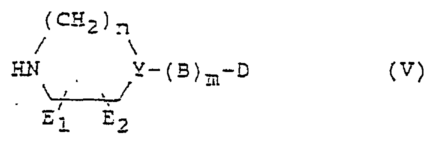 wobei B, D, E₁, E₂, Y, m und n die gleiche Bedeutung haben, wie vorstehend definiert.

12. Verfahren zur Herstellung eines substituierten Benzothiazinderivats nach Anspruch 1, dargestellt durch die nachstehende Formel (Ia): 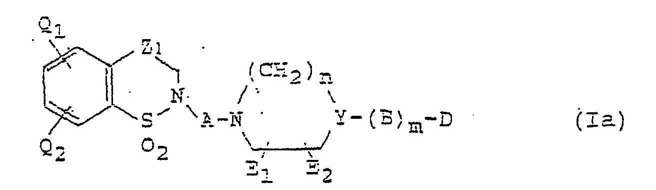 wobei A, B, D, E₁, E₂, Q₁, Q₂, Y, m und n die gleiche Bedeutung haben, wie in Anspruch 1 definiert und Z₁ die gleiche Bedeutung hat, wie in Anspruch 11 definiert, welches umfasst:
Umsetzen einer Verbindung, welche durch die nachstehende Formel (II) dargestellt ist: 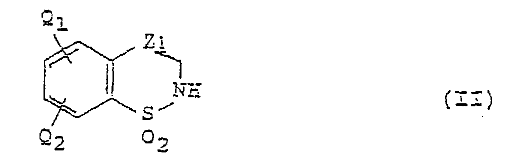 wobei Q₁, Q₂ und Z₁ die gleiche Bedeutung haben, wie vorstehend definiert, mit einer Stickstoff enthaltenden Verbindung, dargestellt durch die nachstehende Formel (VI): 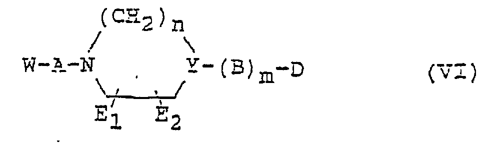 wobei A, B, D, E₁, E₂, Y, m und n die gleiche Bedeutung haben, wie in Anspruch 1 definiert und W die gleiche Bedeutung hat, wie in Anspruch 11 definiert.

13. Verfahren zur Herstellung eines substituierten Benzothiazinderivats nach Anspruch 1, dargestellt durch die nachstehende Formel (Ih): 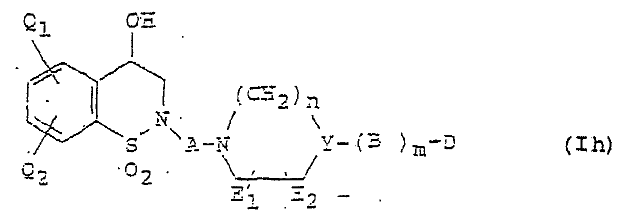 wobei A, B, D, E₁, E₂, Q₁, Q₂, Y, m und n die gleiche Bedeutung haben, wie in Anspruch 1 definiert, welches umfasst:
Reduzieren eines substituierten Benzothiazinderivats, dargestellt durch die nachstehende Formel (Ic): 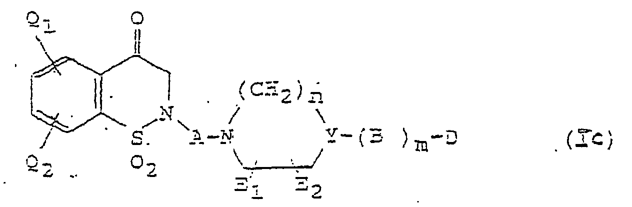 wobei A, B, D, E₁, E₂, Q₁, Q₂, Y, m und n die gleiche Bedeutung haben, wie vorstehend definiert.

14. Verfahren zur Herstellung eines substituierten Benzothiazinderivats nach Anspruch 1, dargestellt durch die nachstehende Formel (Ih): 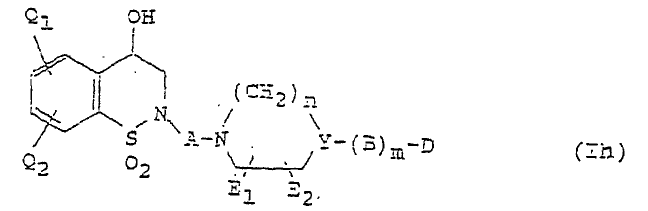 wobei A, B, D, E₁, E₂, Q₁, Q₂, Y, m und n die gleiche Bedeutung haben, wie in Anspruch 1 definiert, welches umfasst:
Reduzieren einer Verbindung, welche durch die nachstehende Formel (VIII) dargestellt ist: 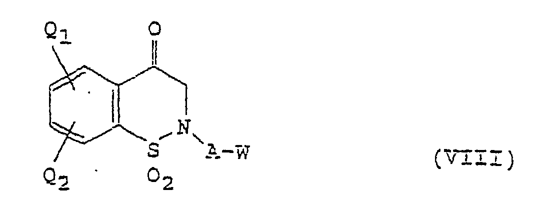 wobei A, Q₁ und Q₂ die gleiche Bedeutung haben, wie in Anspruch 1 definiert und W die gleiche Bedeutung hat, wie in Anspruch 11 definiert, um eine Verbindung, dargestellt durch die nachstehende Formel (X) zu erhalten: 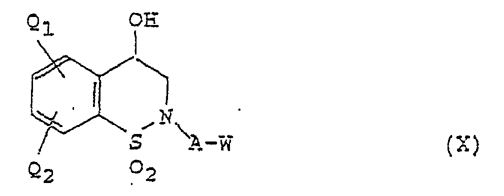 wobei A, Q₁, Q₂ und W die gleiche Bedeutung haben, wie vorstehend definiert, und dann
Umsetzen der erhaltenen Verbindung mit einer Stickstoff enthaltenden, Verbindung, dargestellt durch die nachstehende Formel (V): 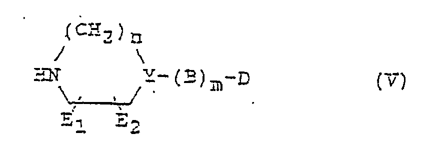 wobei B, D, E₁, E₂, Y, m und n die gleiche Bedeutung haben, wie in Anspruch 1 definiert.

15. Zwischenprodukt, geeignet zur Verwendung zur Herstellung eines Arzneimittels, wobei die Zwischenstufe durch die nachstehende Formel (XX) dargestellt ist: 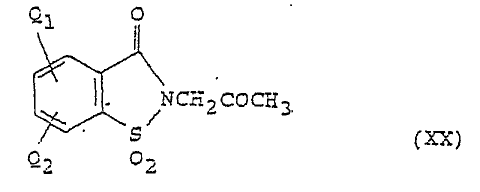 wobei Q₁ und Q₂ die gleiche Bedeutung haben, wie in Anspruch 1 definiert.

16. Zwischenprodukt, geeignet zur Verwendung zur Herstellung eines Arzneimittels, wobei die Zwischenstufe durch die nachstehende Formel (XXI) dargestellt ist: 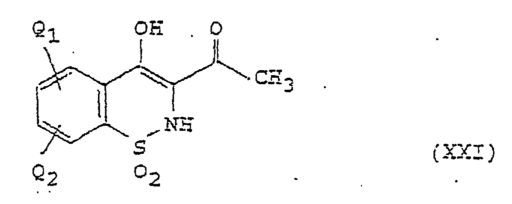 wobei Q₁ und Q₂ die gleiche Bedeutung haben, wie in Anspruch 1 definiert.

17. Zwischenprodukt, geeignet zur Verwendung zur Herstellung eines Arzneimittels, wobei die Zwischenstufe durch die nachstehende Formel (II) dargestellt ist: 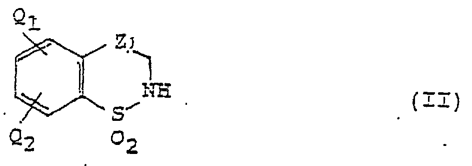 wobei Q₁ und Q₂ die gleiche Bedeutung haben, wie in Anspruch 1 definiert und Z₁ ist: 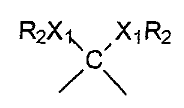 wobei X₁ und R₂ wie in Anspruch 1 definiert sind.

18. Zwischenprodukt, geeignet zur Verwendung zur Herstellung eines Arzneimittels, wobei die Zwischenstufe durch die nachstehende Formel (XXII) dargestellt ist: 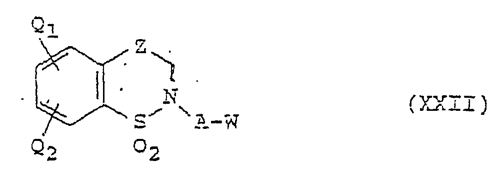 wobei A, Q₁ und Q₂ die gleiche Bedeutung haben, wie in Anspruch 1 definiert, W die gleiche Bedeutung hat, wie in Anspruch 11 definiert und Z ist: 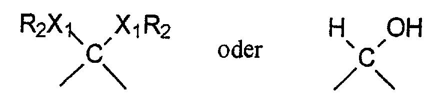 wobei X₁ und R₂ wie in Anspruch 1 definiert sind.

19. Arzneimittel, umfassend als Wirkstoff ein substituiertes Benzothiazinderivat oder ein Salz davon nach einem der Ansprüche 1 bis 10 oder entsprechende Verbindungen, wobei G eine Ethylengruppe ist, falls X₁ und X₂ beides Sauerstoffatome sind.

20. Verwendung eines substituierten Benzothiazinderivats nach einem der Ansprüche 1 bis 10 oder entsprechende Verbindungen, wobei G eine Ethylengruppe ist, falls X₂ und X₃ beides Sauerstoffatome sind, zur Herstellung eines Arzneimittels, welches als Seratonin-2-Rezeptorantagonist fungiert.

21. Verwendung eines substituierten Benzothiazinderivats nach einem der Ansprüche 1 bis 10 oder entsprechende Verbindungen, wobei G eine Ethylengruppe ist, falls X₂ und X₃ beides Sauerstoffatome sind, zur Herstellung eines Arzneimittels, welches als Heilmittel bei Kreislauferkrankungen fungiert.

## Revendications

1. Dérivé substitué de benzothiazine représenté par la formule suivante (I): 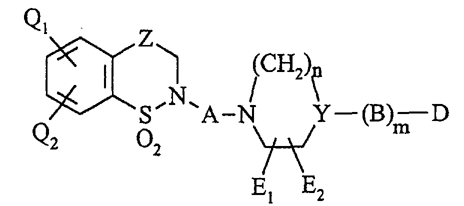 dans laquelle Z représente un des groupes suivants: 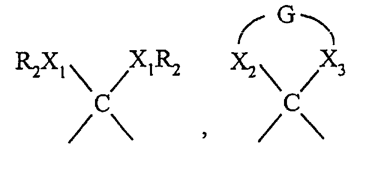 et 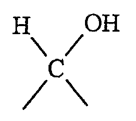 dans lesquels R₂ représente un groupe alkyle en C₁-C₄, un groupe aryle en C₆-C₁₄ ou un groupe aralkyle en C₇-C₂₂, ces groupes étant éventuellement substitués par un ou plusieurs substituants choisis parmi les atomes d'halogène, les groupes alkyle en C₁-C₄ et/ou les groupes alcoxy en C₁-C₄; X₁, X₂ et X₃ représentent chacun indépendamment un atome d'oxygène ou un atome de soufre, G représente un groupe éthylène dont un ou plusieurs des atomes d'hydrogène sont éventuellement remplacés par un nombre égal d'atomes d'halogène et/ou de groupes alkyle en C₁-C₄, aryle en C₆-C₁₄, aralkyle en C₇-C₂₂ et/ou alkylidène en C₁-C₄ ou un groupe triméthylène dont un ou plusieurs des atomes d'hydrogène sont éventuellement remplacés par un nombre égal d'atomes d'halogène et/ou de groupes alkyle en C₁-C₄, aryle en C₆-C₁₄, aralkyle en C₇-C₂₂ et/ou alkylidène en C₁-C₄, à condition que G ne soit pas un groupe éthylène si à la fois X₂ et X₃ sont un atome d'oxygène,
Q₁ représente un atome d'hydrogène, un groupe hydroxy, un atome d'halogène, un groupe alkyle en C₁-C₄, un groupe alcoxy en C₁-C₄,
Q₂ représente un groupe hydroxy, un atome d'halogène, un groupe alkyle en C₁-C₄, un groupe alcoxy en C₁-C₄,
A représente un groupe alkylène en C₂-C₁₀,
Y représente un groupe CH ou un atome d'azote, m représente 0 ou 1, n représente 2 et B représente un groupe carbonyle,
E₁ et E₂ représentent chacun indépendamment un atome d'hydrogène ou un groupe alkyle en C₁₋₄ et D représente un groupe phényle, pyridyle, pyrimidinyle, benzoisothiazolyle, benzisoxazolyle ou indolyle, dans lesquels un ou plusieurs atomes d'hydrogène sont éventuellement remplacés par un atome d'halogène, un groupe alkyle en en C₁₋₄, alcoxy en C₁₋₄, cyano, nitro, carboxyle, (alcoxy en C₁₋₆)carbonyle, (alkyl en C₁₋₄)sulfonylamino, carbamoyle ou hydroxy.

2. Dérivé substitué de benzothiazine ou un de ses sels selon la revendication 1, dans lequel dans la formule (I), Z représente le groupe suivant: 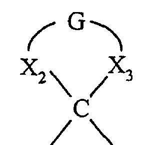 dans lequel G, X₂ et X₃ ont les mêmes significations que définies dans la revendication 1.

3. Dérivé substitué de benzothiazine ou un de ses sels selon la revendication 1, dans lequel dans la formule (I), Z représente le groupe suivant:

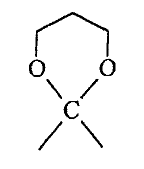

4. Dérivé substitué de benzothiazine ou un de ses sels selon la revendication 1, dans lequel dans la formule (I), Z représente le groupe suivant:

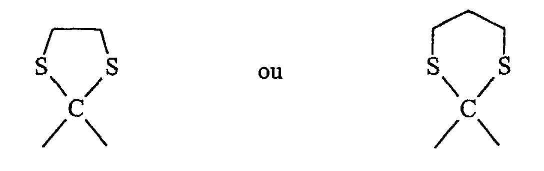

5. Dérivé substitué de benzothiazine ou un de ses sels selon la revendication 1, dans lequel dans la formule (I), Z représente le groupe suivant:

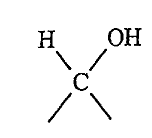

6. Dérivé substitué de benzothiazine ou un de ses sels selon l'une quelconque des revendications 1, 2, 3, 4 et 5, dans lequel dans la formule (I), Q₁ représente un atome d'hydrogène et Q₂ représente un groupe méthoxy.

7. Dérivé substitué de benzothiazine ou un de ses sels selon l'une quelconque des revendications 1, 2, 3, 4, 5 et 6, dans lequel dans la formule (I), A représente un groupe éthylène ou un groupe triméthylène.

8. Dérivé substitué de benzothiazine ou un de ses sels selon l'une quelconque des revendications 1, 2, 3, 4, 5, 6 et 7, dans lequel dans la formule (I), Y représente un groupe CH et D représente un groupe phényle substitué ou non substitué.

9. Dérivé substitué de benzothiazine ou un de ses sels selon l'une quelconque des revendications 1, 2, 3, 4, 5, 6 ou 7, dans lequel dans la formule (I), Y représente un atome d'azote, m représente 0 et D représente un groupe phényle substitué ou non substitué.

10. Dérivé substitué de benzothiazine ou un de ses sels selon l'une quelconque des revendications 1, 2, 3, 4, 5, 6, 7, 8 ou 9, dans lequel dans la formule (I), E₁ et E₂ représentent chacun un atome d'hydrogène.

11. Procédé pour la préparation d'un dérivé substitué de benzothiazine selon la revendication 1, représenté par la formule suivante (Ia): 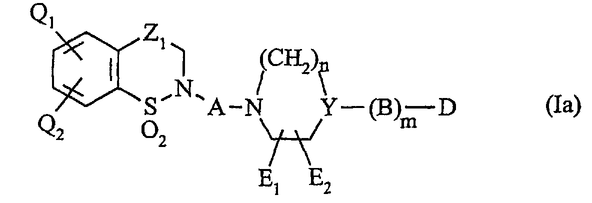 dans laquelle Z₁ représente l'un des groupes suivants: 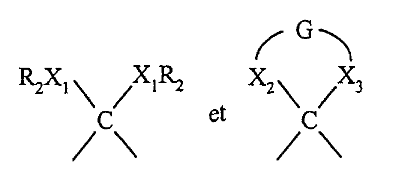 dans lesquels G, R₂, X₁, X₂, X₃, A, B, D, E₁, E₂, Q₁, Q₂, Y, m et n ont les mêmes significations que définies dans la revendication 1, qui comprend:
la réaction d'un composé, qui est représenté par la formule suivante (II): 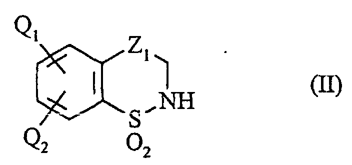 dans laquelle Q₁, Q₂ et Z₁ ont les mêmes significations que définies ci-dessus, avec un composé représenté par la formule suivante (III):
W-A-W' (III)
dans laquelle A a la même signification que définie ci-dessus et W et W' peuvent être identiques ou différents et représentent individuellement un substituant facilement remplaçable par un groupe amino, pour obtenir un composé représenté par la formule suivante (IV): 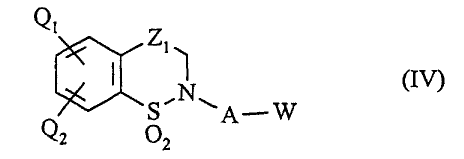 dans laquelle A, Q₁, Q₂, W et Z, ont les mêmes significations que définies ci-dessus; et
ensuite la réaction du composé résultant avec un composé contenant de l'azote représenté par la formule suivante (V): 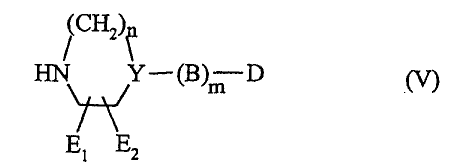 dans laquelle B, D, E₁, E₂, Y, m et n ont les mêmes significations que définies ci-dessus.

12. Procédé pour la préparation d'un dérivé substitué de benzothiazine selon la revendication 1 représenté par la formule suivante (Ia): 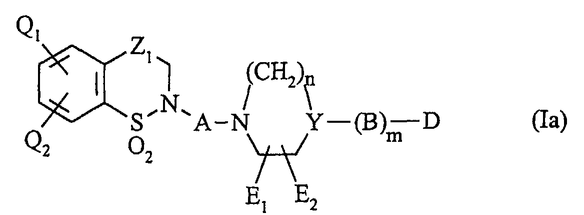 dans laquelle A, B, D, E₁, E₂, Q₁, Q₂, Y, m et n ont les mêmes significations que définies dans la revendication 1 et Z₁ est tel que défini dans la revendication 11, qui comprend:
la réaction d'un composé, qui est représenté par la formule suivante (II): 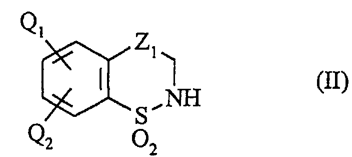 dans laquelle Q₁, Q₂ et Z₁ ont les mêmes significations que définies ci-dessus, avec un composé contenant de l'azote représenté par la formule suivante (VI): 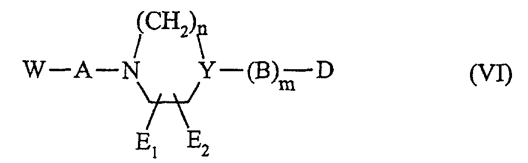 dans laquelle A, B, D, E₁, E₂, Y, m et n ont les mêmes significations que définies dans la revendication 1 et W est tel que défini dans la revendication 11.

13. Procédé pour la préparation d'un dérivé substitué de benzothiazine selon la revendication 1 représenté par la formule suivante (If): 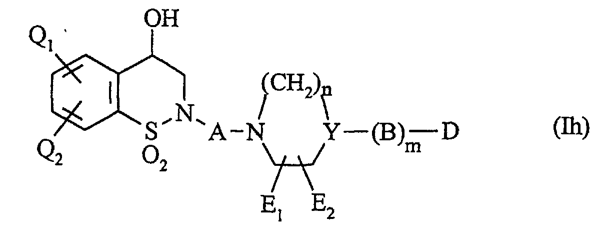 dans laquelle A, B, D, E₁, E₂, Q₁, Q₂, Y, m et n ont les mêmes significations que définies dans la revendication 1, qui comprend le fait de soumettre un dérivé substitué de benzothiazine représenté par la formule suivante (Ic): 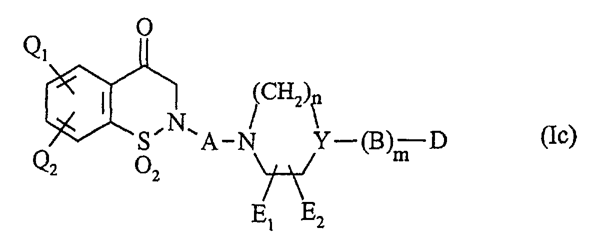 dans laquelle A, B, D, E₁, E₂, Q₁, Q₂, Y, m et n ont les mêmes significations que définies ci-dessus, à une réduction.

14. Procédé pour la préparation d'un dérivé substitué de benzothiazine selon la revendication 1 représenté par la formule suivante (Ih): 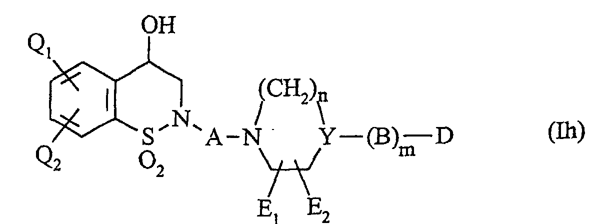 dans laquelle A, B, D, E₁, E₂, Q₁, Q₂, Y, m et n ont les mêmes significations que définies dans la revendication 1, qui comprend le fait de soumettre un composé, qui est représenté par la formule suivante (VIII): 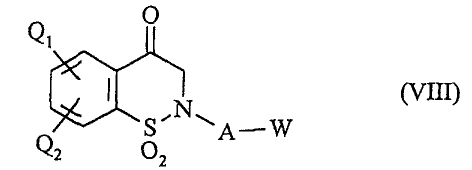 dans laquelle A, Q₁ et Q₂ ont les mêmes significations que définies dans la revendication 1 et W est tel que défini dans la revendication 11, à une réduction pour obtenir un composé représenté par la formule suivante (X): 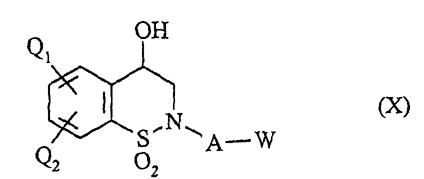 dans laquelle A, Q₁, Q₂ et W ont les mêmes significations que définies ci-dessus, et ensuite
la réaction du composé résultant avec un composé contenant de l'azote représenté par la formule suivante (V): 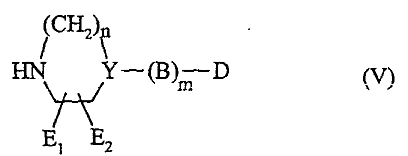 dans laquelle B, D, E₁, E₂, Y, m et n ont les mêmes significations que définies dans la revendication 1.

15. Intermédiaire approprié pour une utilisation dans la production d'un produit pharmaceutique, ledit intermédiaire étant représenté par la formule suivante (XX): 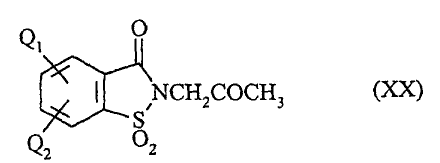 dans laquelle Q₁ et Q₂ ont les mêmes significations que définies dans la revendication 1.

16. Intermédiaire approprié pour une utilisation dans la production d'un produit pharmaceutique, ledit intermédiaire étant représenté par la formule suivante (XXI): 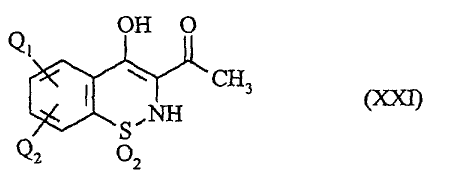 dans laquelle Q₁ et Q₂ ont les mêmes significations que définies dans la revendication 1.

17. Intermédiaire approprié pour une utilisation dans la production d'un produit pharmaceutique, ledit intermédiaire étant représenté par la formule suivante (II): 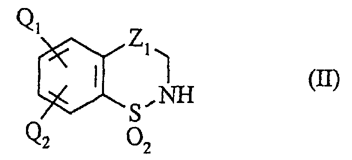 dans laquelle Q₁ et Q₂ ont les mêmes significations que définies dans la revendication 1 et Z₁ est un groupe 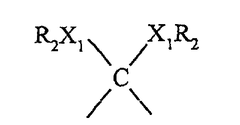 où X₁ et R₂ sont tels que définis dans la revendication 1.

18. Intermédiaire approprié pour une utilisation dans la production d'un produit pharmaceutique, ledit intermédiaire étant représenté par la formule suivante (XXII): 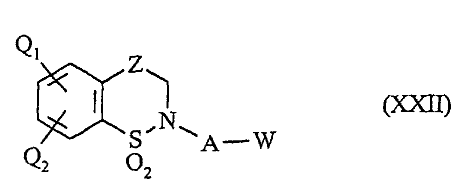 dans laquelle A, Q₁ et Q₂ ont les mêmes significations que définies dans la revendication 1, W est tel que défini dans la revendication 11 et Z est un groupe 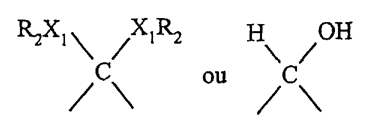 où X₁ et R₂ sont tels que définis dans la revendication 1.

19. Produit pharmaceutique comprenant en tant qu'ingrédient actif un dérivé substitué de benzothiazine ou un de ses sels selon l'une quelconque des revendications 1 à 10 ou les composés respectifs dans lesquels G est un groupe éthylène si à la fois X₂ et X₃ sont un atome d'oxygène.

20. Utilisation d'un dérivé substitué de benzothiazine selon l'une quelconque des revendications 1 à 10 ou des composés respectifs dans lesquels G est un groupe éthylène si à la fois X₂ et X₃ sont un atome d'oxygène pour la préparation d'une composition pharmaceutique agissant en tant qu'antagoniste des récepteurs 2 de la sérotonine.

21. Utilisation d'un dérivé substitué de benzothiazine selon l'une quelconque des revendications 1 à 10 ou des composés respectifs dans lesquels G est un groupe éthylène si à la fois X₂ et X₃ sont un atome d'oxygène pour la préparation d'une composition pharmaceutique agissant en tant qu'agent thérapeutique pour les maladies circulatoires.
